(19) Europäisches Patentamt
European Patent Office
Office européen des brevets

(11) **EP 4 212 880 A1**

(12) **EUROPEAN PATENT APPLICATION**
published in accordance with Art. 153(4) EPC

(43) Date of publication:
**19.07.2023 Bulletin 2023/29**

(21) Application number: **21866778.0**

(22) Date of filing: **08.09.2021**

(51) International Patent Classification (IPC):
***G01N 33/86*** [(2006.01)]

(52) Cooperative Patent Classification (CPC):
**G01N 33/86**

(86) International application number:
**PCT/JP2021/032949**

(87) International publication number:
**WO 2022/054819 (17.03.2022 Gazette 2022/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.09.2020 JP 2020150701**

(71) Applicant: **Sekisui Medical Co., Ltd.**
**Tokyo 103-0027 (JP)**

(72) Inventors:
• **KAWABE, Toshiki**
**Tokyo 103-0027 (JP)**
• **ODA, Yukio**
**Tokyo 103-0027 (JP)**

(74) Representative: **Hartz, Nikolai**
**Wächtershäuser & Hartz**
**Patentanwaltspartnerschaft mbB**
**Weinstrasse 8**
**80333 München (DE)**

(54) **BLOOD COAGULATION REACTION ANALYSIS METHOD**

(57) Provided is a blood coagulation reaction analysis method. The method includes measuring a blood coagulation reaction of a subject specimen and acquiring first data for calculating a blood coagulation time of the subject specimen and second data for estimating a blood coagulation abnormality factor of the subject specimen, wherein the acquiring of the second data includes: obtaining a first derivative $V(i)$ of a coagulation reaction curve $R(i)$; and determining a point $p_k$ where $V(i)$ assumes $X_k$ before reaching a maximum value of $V(i)$, Vmax, and a point $q_k$ where $V(i)$ assumes $X_k$ after reaching Vmax.

Fig. 3

**Description**

Field of the Invention

The present invention relates to a blood coagulation reaction analysis method.

Background of the Invention

A blood coagulation test is a test in which a predetermined reagent is added to a blood specimen of a patient and a blood coagulation time or the like is measured in order to diagnose a blood coagulation ability of the patient. Typical examples of the blood coagulation time include a prothrombin time (PT), an activated partial thromboplastin time (APTT), and a thrombin time. An abnormality in the blood coagulation ability causes a prolongation of coagulation time. Examples of causes of a prolongation of coagulation time include an effect of a coagulation inhibitor drug, a reduction in a component involved in coagulation, a congenital deficiency of a blood coagulation factor, and an acquired appearance of an autoantibody which inhibits a coagulation reaction.

When a prolongation of a coagulation time such as APTT is observed in a blood coagulation test, generally, a cross-mixing test is further performed in order to determine which of a coagulation factor inhibitor (anticoagulation factor), a lupus anticoagulant (LA), and a coagulation factor deficiency such as hemophilia is causing the prolongation of APTT. In the cross-mixing test, with respect to normal plasma, plasma being tested, and mixed plasma containing the plasma being tested and the normal plasma at various capacity ratios, an APTT (immediate reaction) immediately after preparation and an APTT (delayed reaction) after incubating for two hours at 37°C are measured. A measured value of the cross-mixing test is graphically shown with an APTT (seconds) as an axis of ordinate and a capacity ratio between the plasma being tested and the normal plasma as an axis of abscissa. The created graphs of the immediate reaction and the delayed reaction respectively exhibit a pattern which is "convex downward", "straight", or "convex upward" in accordance with an APTT prolongation factor. The APTT prolongation factor is determined based on the patterns of the immediate reaction and the delayed reaction.

In a blood coagulation test, a coagulation reaction curve can be obtained by measuring, over time, a blood coagulation reaction amount after adding a reagent to the blood specimen. The coagulation reaction curve has different shapes in accordance with a type of abnormality of a blood coagulation system (Non Patent Literature 1). Therefore, methods of determining an abnormality of the blood coagulation system based on the coagulation reaction curve are disclosed. For example, Patent Literature 1 to 3 and Non Patent Literature 2 to 4 describe methods of assessing a presence or absence of an abnormality of a coagulation factor in a patient based on parameters related to a primary differential curve and a quadratic differential curve of a coagulation reaction curve with respect to blood of the patient such as a maximum coagulation rate, a maximum coagulation acceleration, a maximum coagulation deceleration, and times required to reach the maximum coagulation rate, the maximum coagulation acceleration, and the maximum coagulation deceleration. Patent Literature 4 describes a method of determining a degree of severity of hemophilia based on an average rate of change in a coagulation rate during a period in which a coagulation reaction of a patient reaches a maximum coagulation rate or a maximum coagulation acceleration. Patent Literature 5 and 6 describe methods of calculating a peak width at a predetermined height of a coagulation reaction rate curve and determining a presence or absence of an abnormality of a coagulation factor, a concentration of the coagulation factor, a coagulation time prolongation factor, or the like using information based on the peak width.

[Citation List]

[Patent Literature]

[Patent Literature 1] JP-A-2016-194426
[Patent Literature 2] JP-A-2016-118442
[Patent Literature 3] JP-A-2017-106925
[Patent Literature 4] JP-A-2018-017619
[Patent Literature 5] JP-A-2019-086517
[Patent Literature 6] WO 2020/101025

[Non Patent Literature]

[Non Patent Literature 1] British Journal of Haematology, 1997, 98:68-73

[Non Patent Literature 2] The Japanese Journal of Clinical Hematology, 2017, vol. 58, no. 9, p. 1754, PS2-37-7

[Non Patent Literature 3] The Japanese Journal of Thrombosis and Hemostasis, 2018, vol. 29, no. 2, p. 184, 0-056, P-080

[Non Patent Literature 4] The Japanese Journal of Thrombosis and Hemostasis, 2018, vol. 29, no. 4, p. 413-420

Summary of the Invention

The present invention relates to a blood coagulation reaction analysis method which enables data for measuring a coagulation time of a blood specimen and data useful for estimating a coagulation abnormality factor in the blood specimen to be acquired.

Specifically, the present invention provides the following.

[1] A blood coagulation reaction analysis method, comprising:

measuring a blood coagulation reaction of a subject specimen and acquiring first data for calculating a blood coagulation time of the subject specimen and second data for estimating a blood coagulation abnormality factor of the subject specimen, wherein
the acquiring of the second data comprises:

obtaining a first derivative $V(i)$ of a coagulation reaction curve $R(i)$, where i represents a number of measurement points or time; and
determining a point $p_k$ where $V(i)$ assumes $X_k$ before reaching a maximum value of $V(i)$, Vmax, and a point $q_k$ where $V(i)$ assumes $X_k$ after reaching Vmax, where k represents a series of integers from 1 to n, n denotes an integer equal to or larger than 2, and $0 < X_k < $ Vmax.

[2] The method according to [1], wherein the $X_k$ is specified by Vmax $\times$ $S_k$% (wherein $S_k$ ranges from 0.5 to 99) .

[3] The method according to [1] or [2], wherein the acquiring of the second data further comprises calculating $p_k$ or $q_k$ or statistics thereof as the second data.

[4] The method according to [1] or [2], wherein the acquiring of the second data further comprises calculating, as the second data, at least one selected from the group consisting of a pre-Ave, a post-Ave, a pre-SD, a post-SD, a pre-CV, a post-CV, a pre-post average difference, a pre-post SD ratio, $M_k$, $W_k$, a distortion index, and a peakedness index,

the pre-Ave, the pre-SD, and the pre-CV respectively representing an average value, a standard deviation, and a coefficient of variation of $p_k$,
the post-Ave, the post-SD, and the post-CV respectively representing an average value, a standard deviation, and a coefficient of variation of $q_k$,
the pre-post average difference representing (post-Ave - pre-Ave)/(average value of $p_k$ and $q_k$),
the pre-post SD ratio representing post-SD/pre-SD,
$M_k$ representing $(p_k + q_k)/2$,
$W_k$ representing $q_k - p_k$,
the distortion index representing a coefficient of variation of $M_k$, and
the peakedness index representing (sum or average value of $W_k$ with respect to lower part of peak of $V(i)$(sum or average value of $W_k$ with respect to upper part of peak of $V(i)$).

[5] The method according to [4], wherein the acquiring of the second data further comprises calculating, as the second data, a standard deviation interval (SDI) of an objective parameter with respect to the subject specimen, where

SDI of objective parameter with respect to the subject specimen = $(\alpha - \beta) \div \gamma$, wherein
$\alpha$: value of objective parameter from subject specimen
$\beta$: reference value of value of objective parameter based on data of normal specimen group
$\gamma$: reference deviation of value of objective parameter based on data of normal specimen group, and
the objective parameter is any two selected from the group consisting of the pre-CV, the post-CV, the distortion index, the peakedness index, the pre-post average difference, and the pre-post SD ratio.

[6] The method according to any one of [1] to [5], further comprising calculating a blood coagulation time using the first data.

[7] The method according to any one of [1] to [6], wherein the first data comprises a point R(E) (where E denotes a coagulation reaction end point) on the coagulation reaction curve R(i) or a maximum value of V(i), Vmax.

[8] The method according to any one of [1] to [7], comprising acquiring the second data after continuing measurement of the blood coagulation reaction until an end of the coagulation reaction.

[9] The method according to any one of [1] to [8], further comprising performing estimation of a blood coagulation abnormality factor of the subject specimen based on the second data.

[10] The method according to [9], wherein the estimation of the blood coagulation abnormality factor comprises estimating a type of the blood coagulation abnormality factor of the subject specimen, and the type of the blood coagulation abnormality factor is selected from the group consisting of a coagulation factor deficiency, lupus anticoagulant-positive, a coagulation factor inhibitor, and heparin-positive.

[11] The method according to [9] or [10], wherein the estimation of the blood coagulation abnormality factor comprises estimating a presence or absence of the blood coagulation abnormality factor of the subject specimen.

[12] The method according to any one of [9] to [11], wherein the estimation of the blood coagulation abnormality factor comprises estimating the blood coagulation abnormality factor of the subject specimen in accordance with an estimation model constructed by machine learning,

> the estimation model is constructed by machine learning which uses a feature amount representing a blood coagulation reaction of each specimen in a supervised specimen group as an explanatory variable and which uses data with respect to a presence or absence of a coagulation abnormality or to a coagulation abnormality factor of each specimen in the supervised specimen group as a target variable,
> the supervised specimen group comprises a blood specimen without a coagulation abnormality and blood specimens with respectively different coagulation abnormality factors,
> the feature amount comprises the second data, and
> the estimation model estimates a presence or absence of a coagulation abnormality or estimates a coagulation abnormality factor of the subject specimen from the feature amount of the subject specimen.

Advantageous Effects of Invention

The method according to the present invention enables data for measuring a coagulation time of a blood specimen and data useful for estimating a coagulation abnormality factor in the blood specimen to be acquired. According to the present invention, not only can a presence or absence of a coagulation abnormality factor in a blood specimen be estimated but a coagulation abnormality factor in an abnormal specimen can be estimated without having to perform a time-consuming cross-mixing test as was conventional. In addition, according to the present invention, an apparently normal specimen of which a coagulation time is not prolonged but which actually has a coagulation abnormality factor can be detected by a simple procedure.

Brief Description of the Drawings

[Figure 1] Figure 1 shows an embodiment of a procedure of a blood coagulation reaction analysis method according to the present invention.

[Figure 2] Figure 2 shows an example of coagulation reaction measured data.

[Figure 3] Figure 3 is a conceptual diagram for explaining $X_k$, $p_k$, and $q_k$.

[Figure 4] Figure 4 shows a coagulation reaction curve R (above) and a first derivative V (below) of various specimens. A circular mark and a triangular mark on V respectively represent $(p_k, X_k)$ and $(q_k, X_k)$. A dotted line represents a line connecting $(M_k, X_k)$. FVIII: FVIII-deficient specimen, FIX: FIX-deficient specimen, heparin: heparin-positive specimen, inhibitor: inhibitor-positive specimen, LA: LA-positive specimen.

[Figure 5] Figure 5A shows the first derivative V of an FVIII-deficient specimen. Meanings of a circular mark, a triangular mark, and a dotted line are the same as in Figure 4. Figure 5B is a chart in which $p_k$, $q_k$, and $M_k$ in Figure 5A are plotted against time. Figure 5C is a chart representing a change in $W_k$ obtained from $p_k$ and $q_k$ in Figure 5A due to k.

[Figure 6] Figure 6A shows the first derivative V of a normal specimen. Meanings of a circular mark, a triangular mark, and a dotted line are the same as in Figure 4. Figure 6B is a chart in which $p_k$, $q_k$, and $M_k$ in Figure 6A are plotted against time. Figure 6C is a chart representing a change in $W_k$ obtained from $p_k$ and $q_k$ in Figure 6A due to k.

[Figure 7] Figure 7 is a conceptual diagram showing a configuration of an automated analyzer for carrying out the blood coagulation reaction analysis method according to the present invention.

[Figure 8] Figure 8 shows an APTT of various specimens measured in a first example.

[Figure 9] Figure 9A is a two-dimensional plot of a pre-CV and a post-CV of a specimen. Figure 9B is a two-dimensional plot of relative_pre-CV and relative_post-CV of a specimen. Figure 9C is a two-dimensional plot of an SDI of the pre-CV and an SDI of the post-CV of a specimen. Heparin: specimen of heparin concentration series, FIX: specimen of FIX activity series, FVIII: specimen of FVIII activity series, VIII#Inh: FVIII inhibitor specimen, LA: LA-positive specimen, and PNP: normal specimen.

[Figure 10] Figure 10 shows a relationship between factor activity (logarithmic value) and relative_pre-CV (A), relative_post-CV (B), and a distance (C) from an origin on a plot shown in Figure 9B. Displayed specimen types are the same as in Figure 9.

[Figure 11] Figure 11A is a two-dimensional plot of a distortion index and a peakedness index of a specimen. Figure 11B is a two-dimensional plot of relative_distortion index and relative_peakedness index of a specimen. Figure 11C is a two-dimensional plot of an SDI of the distortion index and an SDI of the peakedness index of a specimen. Displayed specimen types are the same as in Figure 9.

[Figure 12] Figure 12 shows a relationship between factor activity (logarithmic value) and relative_distortion index (A), relative_peakedness index (B), and a distance (C) from an origin on a plot shown in Figure 11B. Displayed specimen types are the same as in Figure 9.

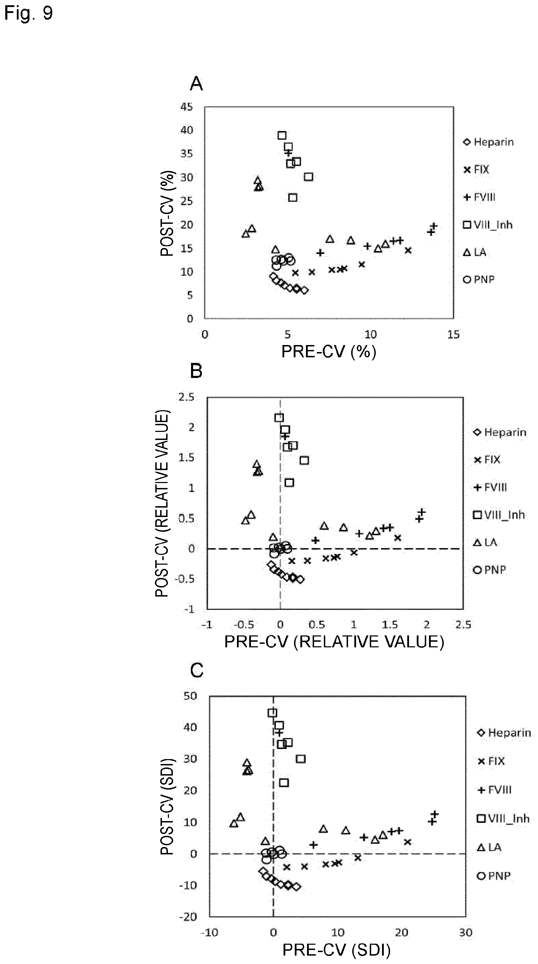

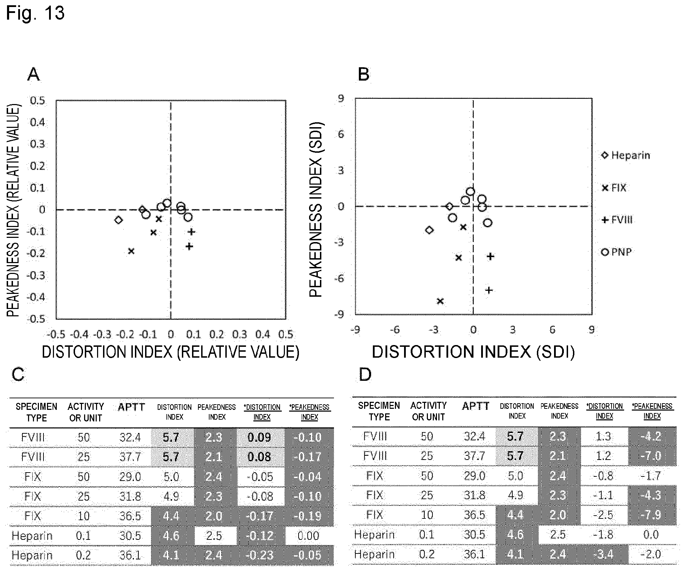

[Figure 13] Figure 13A is a two-dimensional plot of relative_distortion index and relative_peakedness index of an abnormal specimen which has a coagulation abnormality factor but of which an APTT is within a normal range. Displayed specimen types are the same as in Figure 9. Figure 13B is a two-dimensional plot of a distortion index SDI and a peakedness index SDI of the same abnormal specimen. Figure 13C is a table showing an APTT, a distortion index, a peakedness index, relative_distortion index, and relative_peakedness index of the abnormal specimen shown in Figure 13A. Figure 13D is a table showing an APTT, a distortion index, a peakedness index, a distortion index SDI, and a peakedness index SDI of the abnormal specimen shown in Figure 13B.

[Figure 14] Figure 14A is a two-dimensional plot of a pre-post average difference and a pre-post SD ratio of a specimen. Figure 14B is a two-dimensional plot of relative_pre-post average difference and relative_pre-post SD ratio of a specimen. Figure 14C is a two-dimensional plot of an SDI of the pre-post average difference and an SDI of the pre-post SD ratio of a specimen. Displayed specimen types are the same as in Figure 9.

[Figure 15] Figure 15 shows a relationship between factor activity (logarithmic value) and relative_pre-post average difference (A), relative_pre-post SD ratio (B), and a distance (C) from an origin on a plot shown in Figure 14B. Displayed specimen types are the same as in Figure 9.

[Figure 16] Figure 16 shows a distribution area of a plot of each specimen type on the two-dimensional plot shown in Figure 9C. Heparin: specimen of heparin concentration series, FIX: specimen of FIX activity series, FVIII: specimen of FVIII activity series, VIII#Inh: FVIII inhibitor specimen, LA: LA-positive specimen, and PNP: normal specimen.

[Figure 17] Figure 17 shows a distribution area of a plot of each specimen type on the two-dimensional plot shown in Figure 11C. Displayed specimen types are the same as in Figure 16.

[Figure 18] Figure 18 shows a distribution area of a plot of each specimen type on the two-dimensional plot shown in Figure 14C. Displayed specimen types are the same as in Figure 16.

[Figure 19] Figure 19 is a two-dimensional plot of relative_VmaxT and relative_Vmax of a specimen. Displayed specimen types are the same as in Figure 9.

Detailed Description of the Invention

In a blood coagulation test, a predetermined reagent is added to a blood specimen, a subsequent blood coagulation reaction is measured, and a blood coagulation time is measured from the coagulation reaction. In the present specification described below, a blood specimen may be simply referred to as a specimen. In the measurement of the blood coagulation reaction, general means such as optical means which measures a scattered light quantity, transmittance, absorbance, or the like, mechanical means which measures a viscosity of blood plasma, or the like is used. A blood coagulation reaction is generally represented by a coagulation reaction curve indicating a change in an amount of a coagulation reaction over time. Although dependent on measuring means, the coagulation reaction curve of a normal specimen without a coagulation abnormality factor basically exhibits a sigmoidal shape. For example, the coagulation reaction curve of a normal specimen based on a scattered light quantity usually rises abruptly as coagulation progresses at a time point where a certain amount of time has elapsed from the addition of a reagent and, subsequently, reaches a plateau as the coagulation reaction nears its end. On the other hand, the coagulation reaction curve of an abnormal specimen with a coagulation abnormality factor exhibits various shapes dependent on a cause of the abnormality such as a delayed rise time or a gradual rise of the curve.

In the measurement of a blood coagulation time of a specimen, data can be collected until the end of the coagulation reaction or, in other words, until the coagulation reaction curve reaches a plateau, and a coagulation time can be calculated based on the data. For example, if a reaction amount from a start of a reaction to an end of the reaction is taken as 100%, a time until the reaction amount reaches 50% can be calculated as a coagulation time. Alternatively, the coagulation time can be calculated based on a rate of change of the coagulation reaction curve such as a change over time of an integrated value of a coagulation reaction at a peak or during a small time slot of a coagulation reaction rate (refer to JP-A-6-249855). With the latter method, since the coagulation time can be calculated before the end of the coagulation reaction, the coagulation time can be measured in a shorter period of time. With specimens having a coagulation abnormality factor, in many cases, the coagulation time is prolonged as compared to normal specimens. The prolongation of the coagulation time is an indicator of a presence or absence of a coagulation abnormality factor. On the other hand, a type of the coagulation abnormality factor (a prolongation factor of coagulation time) cannot be estimated from the coagulation time.

Conventionally, determination of a prolongation factor of coagulation time (a type of a coagulation abnormality factor) is mainly performed by a cross-mixing test. Therefore, with conventional methods, a prolongation factor of a coagulation time cannot be determined unless a cross-mixing test is performed separately from a coagulation time measurement. Furthermore, a cross-mixing test requires measurements of an immediate reaction and a delayed reaction after a two-hour incubation with respect to a mixed specimen of a subject specimen and a normal specimen and, therefore, takes time and effort.

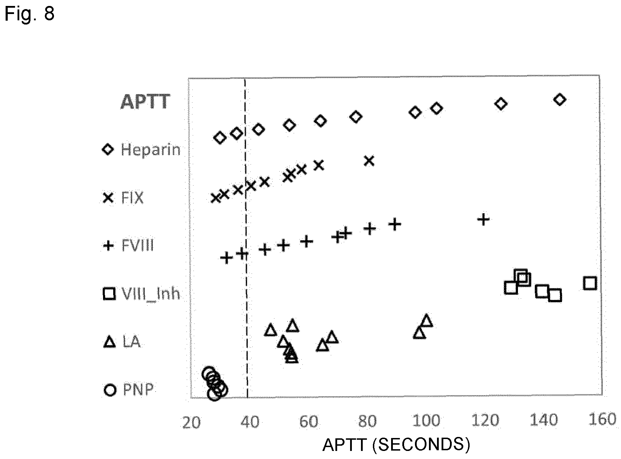

In addition, with conventional methods, a cross-mixing test is normally only applied to specimens of which a prolongation of a coagulation time is observed in a coagulation reaction measurement and, meanwhile, a specimen of which a coagulation time is within a normal range is conventionally considered a normal specimen. However, a study conducted by the present inventors has revealed that a specimen which actually has a coagulation abnormality factor may exhibit a coagulation time within a normal range due to the specimen being highly responsive to a reagent for coagulation measurement or the like (refer to Figure 8). Since such a specimen which actually has an abnormality factor but which does not exhibit an apparent prolongation of a coagulation time is not subjected to a cross-mixing test and other tests in conventional methods, the specimen is not detected as an abnormal specimen.

In the present invention, during a coagulation reaction measurement, both data for calculating a coagulation time and data for estimating a coagulation abnormality factor are acquired. Therefore, the present invention enables both a coagulation time of a specimen and data for estimating a coagulation abnormality factor of the specimen to be acquired in one measurement. In addition, the present invention enables a coagulation abnormality factor to be estimated or data for estimating the coagulation abnormality factor to be acquired during a coagulation reaction measurement without having to perform a time-consuming cross-mixing test. Furthermore, according to the present invention, a specimen which does not exhibit a prolongation of a coagulation time but which actually has a coagulation abnormality factor can be detected by a simple procedure.

[Blood coagulation reaction analysis method]

The present invention provides a blood coagulation reaction analysis method. In the blood coagulation reaction analysis method according to the present invention (hereinafter, also referred to as a method according to the present invention), a blood coagulation reaction of a blood specimen under test (hereinafter, also referred to as a subject specimen) is measured and, based on time-series data of a coagulation reaction obtained by the measurement, data for calculating a blood coagulation time of the subject specimen (first data) and data for estimating a blood coagulation abnormality factor of the subject specimen (second data) are acquired.

Examples of the blood coagulation time which can be calculated in accordance with the present invention include a prothrombin time (PT), an activated partial thromboplastin time (APTT), and a coagulation time in fibrinogen (Fbg) concentration measurement. In the present specification presented below, the method according to the present invention will be described by mainly taking an activated partial thromboplastin time (APTT) as an example of a coagulation time. Any person skilled in the art can modify the method according to the present invention to other coagulation times (for example, a prothrombin time (PT)).

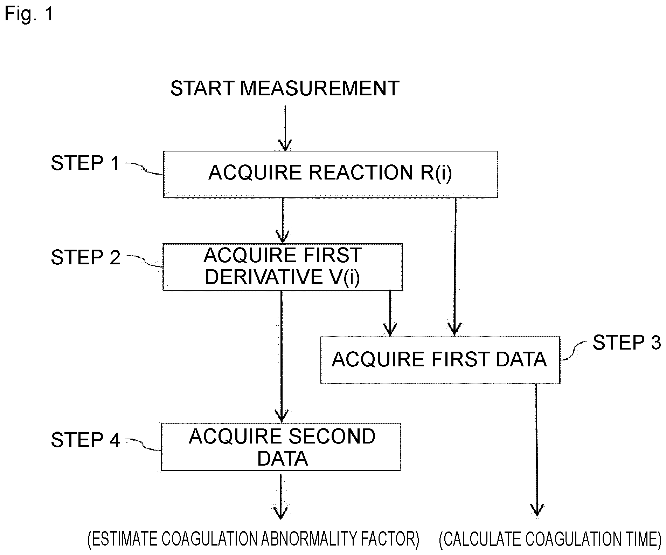

Hereinafter, the method according to the present invention will be described with reference to a basic flow of an embodiment of the method according to the present invention shown in Figure 1.

1. Coagulation reaction measurement

In the method according to the present invention, blood plasma of a subject being tested is preferably used as a subject specimen. An anticoagulant normally used in a coagulation test can be added to the specimen. For example, blood plasma is obtained by collecting blood using a blood collection tube with sodium citrate and subsequently subjecting the collected blood to centrifugal separation.

In a measurement of a blood coagulation reaction, a reagent for coagulation time measurement is added to the subject specimen to start a blood coagulation reaction. A coagulation reaction of a mixed liquid containing the reagent and the subject specimen can be measured. The reagent for coagulation time measurement to be used can be optionally selected according to the purpose of measurement. Reagents for measuring various coagulation times are commercially available (for example, APTT Reagent Coagpia APTT-N manufactured by SEKISUI MEDICAL CO., LTD.). In the measurement of a coagulation reaction, general means such as optical means which measures a scattered light quantity, transmittance, absorbance, or the like, mechanical means which measures a viscosity of blood plasma, or the like may be used. In the present specification presented below, the method according to the present invention will be described using a coagulation reaction measurement based on a scattered light quantity as an example.

While a reaction start time point of a coagulation reaction can be typically defined as a time point where a reagent is mixed with a specimen and the coagulation reaction is started, other timings may be defined as the reaction start time point. A period of time during which the measurement of the coagulation reaction is continued can be, for example, several ten seconds to around seven minutes from the time point at which the reagent is mixed with the specimen. While the measurement time may be an optionally-set fixed value, the measurement time may conclude at a time point where an end of the coagulation reaction of each specimen is detected. During the measurement time, a measurement (photometry in a case where detection is performed optically) of a progress of the coagulation reaction can be repetitively performed at predetermined intervals. For example, measurements may be performed at 0.1-second intervals. A temperature of the mixed liquid during the measurement corresponds to normal conditions such as 30°C or higher and 40°C or lower and, preferably, 35°C or higher and 39°C or lower. In addition, various conditions of measurement can be appropriately set in accordance with the subject specimen, the reagent, the measurement means, and the like.

A series of operations in the coagulation reaction measurement described above can be performed using an automated analyzer. An example of the automated analyzer is the Automated Coagulation Analyzer CP3000 (manufactured by SEKISUI MEDICAL CO., LTD.). Alternatively, a part of the operations may be performed manually. For example, the subject specimen can be prepared by hand and subsequent operations can be performed by an automated analyzer.

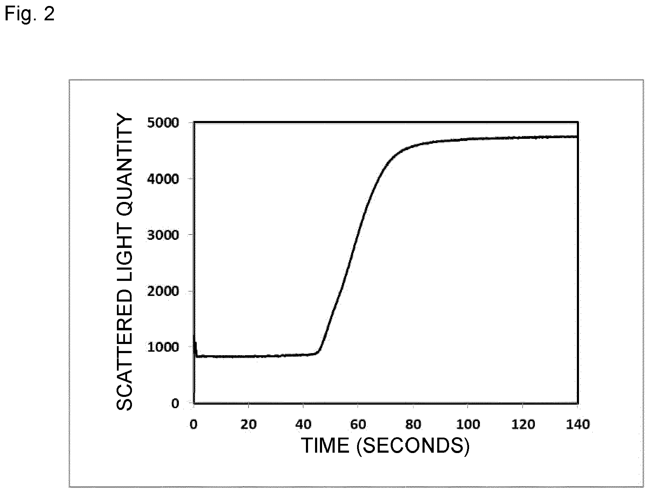

Due to the coagulation reaction measurement described above, measured data D(i) (a photometric value of a scattered light quantity) is sequentially acquired. In this case, "i" represents the number of measurement points or a time from start of the coagulation reaction (also simply referred to as time). For example, when the measurement (photometry) interval is 0.1 seconds, "i" is represented as time = 0.1 × number of measurement points. Figure 2 shows an example of measured data. In Figure 2, an axis of abscissa represents time and an axis of ordinate represents scattered light quantity. Since the coagulation reaction of the mixed liquid progresses as time elapses, the scattered light quantity increases. A coagulation reaction curve based on the scattered light quantity such as that shown in Figure 2 usually assumes a sigmoidal shape.

2. Acquisition of reaction R(i) and first derivative V(i)

Next, a reaction R(i) is acquired from the measured data D(i) (step 1). Since the measured data D(i) contains noise during photometry and fluctuations which appear immediately after start of photometry and which are unrelated to the reaction, the measured value is preferably subjected to smoothing by known methods. In addition, when a coagulation reaction is photometrically measured based on a scattered light quantity, zero point adjustment processing of subtracting a scattered light quantity derived from the mixed specimen liquid prior to the reaction is preferably performed. Any of various known methods related to denoising can be used in the smoothing of measured data. For example, examples of smoothing include filtering and processing in which a difference value or a derivative value is obtained by an operation such as an intra-section average gradient to be described later and subsequently integrating the difference value or the derivative value. In zero point adjustment, for example, smoothed measured data may be adjusted so as to assume a value of 0 at a measurement start time point. Furthermore, initial fluctuation removal may be performed with respect to the measured data D(i). The initial fluctuation removal may be performed such that all values from the measurement start time point until an initial fluctuation removal time determined in advance become zero. Preferably, the measured data D(i) is subjected to smoothing or zero point adjustment to acquire reaction R(i). More preferably, the measured data D(i) is subjected to smoothing and zero point adjustment to acquire the reaction R(i). Alternatively, after the measured data D(i) is subjected to smoothing and zero point adjustment, the measured data D(i) is further converted into a relative value to acquire the reaction R(i). For example, the reaction R(i) may be acquired by converting D(i) so that D(i) at the measurement start time point is 0 and D(i) at the coagulation reaction end point is a predetermined value such as 100. The reaction R(i) constitutes a coagulation reaction curve.

A first derivative V(i) thereof is acquired from the obtained reaction R(i) (step 2). Differential processing of obtaining V(i) from R(i) can be performed by any method such as calculating an intra-section average gradient value. In the calculation of an intra-section average gradient value, a certain number of measurement points before and after each measurement point i such as 2K+1-number of measurement points from i-K to i+K can be used, where K denotes any integer. For example, when K is 2, five measurement points of i-2, i-1, i, i+1, and i+2 can be used. An average gradient value refers to a gradient value when linearly approximating the plurality of measurement points. Conventional methods such as a least-squares method can be used as a method to calculate linear approximation. An average gradient value of the measurement points can be considered a first derivative at the measurement point i. Furthermore, a relative value of the first derivative of R(i) may be acquired as V(i). For example, V(i) may be acquired by converting a first derivative value of R(i) so that a value at the measurement start time point is 0 and a maximum value is a predetermined value such as 100. The first derivative V(i) constitutes a curve representing a rate or a rate of change of a coagulation reaction.

From R(i) or V(i) obtained as described above, data (first data) for calculating a blood coagulation time of a subject specimen and data (second data) for estimating a blood coagulation abnormality factor of the subject specimen are acquired. In the method according to the present invention, acquisition of R(i) and V(i) with respect to a subject specimen may be performed in parallel with a measurement of a coagulation reaction of the subject specimen or performed after the measurement of a coagulation reaction ends. In the method according to the present invention, the measurement of the coagulation reaction of the subject specimen is preferably performed until an end of the coagulation reaction. The end of the coagulation reaction can be determined in accordance with any criterion such as a time point where R(i) reaches a plateau or a time point where, after reaching a peak, V(i) decreases to 0 or a constant value (for example, S% or less of a maximum peak).

As described above, each of D(i), R(i), and V(i) in the present specification may be a function of the number of measurement points or a function of time. The first data and the second data to be described later may also be data based on the number of measurement points or data based on time. In the present specification described below, R(i) and V(i) may be respectively abbreviated as simply R and V.

In an embodiment, R and V with respect to a subject specimen are sequentially acquired while performing a coagulation reaction measurement of the subject specimen. At an appropriate time during the coagulation reaction measurement, the first data is acquired from R or V and further, when necessary, a coagulation time of the subject specimen is acquired from the first data. Subsequently, preferably after continuously acquiring R and V until the coagulation reaction ends, the second data is acquired and further, when necessary, a coagulation abnormality factor of the subject specimen is estimated from the second data.

In another embodiment, R with respect to a subject specimen is sequentially acquired while performing a coagulation reaction measurement of the subject specimen. At an appropriate time during the coagulation reaction measurement, the first data is acquired from the R and further, when necessary, a coagulation time of the subject specimen is acquired from the first data. Subsequently, preferably after continuously acquiring R until the coagulation reaction ends, V is acquired from the R, the second data is acquired and further, when necessary, a coagulation abnormality factor of the subject specimen is estimated from the second data.

In another embodiment, R with respect to a subject specimen is preferably sequentially acquired while performing a coagulation reaction measurement of the subject specimen until the end of the coagulation reaction. Subsequently, V, first data, and second data are acquired. Furthermore, when necessary, a coagulation time of the subject specimen is calculated from the first data and a coagulation abnormality factor of the subject specimen is estimated from the second data.

In another embodiment, preferably, after continuing a coagulation reaction measurement of a subject specimen until the end of the coagulation reaction, R and V are acquired and first data and second data are acquired. Furthermore, when necessary, a coagulation time of the subject specimen is calculated from the first data and a coagulation abnormality factor of the subject specimen is estimated from the second data.

In each of the embodiments described above, the coagulation reaction measurement may be ended at a timing preceding the end of the coagulation reaction as long as measured values necessary for acquiring the first data and the second data have already been obtained.

3. Acquisition of first data

In the method according to the present invention, acquisition of first data and calculation of a coagulation time of a subject specimen using the first data (step 3) can be performed in accordance with any method. Examples of a method of calculating a coagulation time include: a method of calculating, as a coagulation time, a time point where R(i) reaches N% (where N is any value, the same applies hereinafter) of R(E) representing a reaction R at a coagulation reaction end point E; a method of calculating, as a coagulation time, a time point where V(i) reaches a maximum value Vmax or N% thereof; a method of calculating a coagulation time based on a change over time of an integrated value of R(i) in a small time slot (refer to JP-A-6-249855 and Japanese Patent Application No. 2019-237427); a method of calculating a coagulation time based on a weighted average time of V(i) (refer to Japanese Patent Application No. 2020-039344); and a method of calculating, as a coagulation time, using a time point where by taking a time point where V(i) reaches a predetermined value after first reaching the maximum value Vmax as an origin of calculation Te, a time point where R(i) reaches N% of R(Te) (refer to Japanese Patent Application No. 2020-068877).

Therefore, when the methods of calculating a coagulation time cited above are to be used, examples of the first data acquired in the method according to the present invention include: data related to R(E) representing a reaction R at a coagulation reaction end point E or a time point where R(i) reaches N% of R(E); data related to the maximum value Vmax of V(i) or a time point where V(i) reaches Vmax or N% thereof; data related to a change over time of an integrated value of R(i) in a small time slot; a weighted average time of V(i); and data related to an origin of calculation Te representing a point where V(i) reaches a predetermined value after first reaching Vmax or a time point where R(i) reaches N% of R(Te). However, methods of calculating a coagulation time in the method according to the present invention and types of the first data used in the calculation methods are not limited to the above.

4. Acquisition of second data

In the method according to the present invention, a first derivative V is used in acquisition of second data and estimation of a coagulation abnormality factor of a subject specimen using the second data (step 4). As described above, V may be sequentially acquired while performing a coagulation reaction measurement or acquired after the coagulation reaction measurement ends. Preferably, in the method according to the present invention, after performing a coagulation reaction measurement until the end of a coagulation reaction, the second data is acquired using the acquired V and, when necessary, estimation of a coagulation abnormality factor is further performed. Alternatively, as long as V necessary for acquiring the second data has already been obtained, the coagulation reaction measurement may be ended at a timing preceding the end of the coagulation reaction.

In a procedure of acquiring the second data, the number of measurement points or a time i where V assumes a reference value $X_k$ set in advance (in other words, where $V(i) = X_k$ is satisfied) is determined. Alternatively, when $V(i) < X_k < V(i+1)$ or $V(i-1) < X_k < V(i)$, i can be selected as the number of measurement points or a time satisfying $V(i) = X_k$. Since V usually has a peak shape with a maximum value Vmax as a peak top, at least one i satisfying $V(i) = X_k$ exists in each of time points before and after V reaches Vmax. In the method according to the present invention, i satisfying $V(i) = X_k$ is respectively determined with respect to V before and after reaching Vmax. Furthermore, in the method according to the present invention, $X_k$ is a variable and i corresponding to each $X_k$ is determined. For example, when there are n-number of $X_k$ expressed as $(X_1, ..., X_n)$ (where k represents a series of integers from 1 to n and n represents an integer equal to or larger than 2), a point $p_k$ where V(i) assumes $X_k$ before reaching Vmax and a point $q_k$ where V(i) assumes $X_k$ after reaching Vmax are respectively determined and n-number of $p_k$ ($p_1, . . . , p_n$) and n-number of $q_k$ ($q_1, ..., q_n$) are obtained. While a point where V assumes $X_k$ after reaching Vmax may be detected in plurality in a case where V has a plurality of peaks, in such a case, a maximum point among the detected points is selected as $q_k$. In a similar manner, when a point where V assumes $X_k$ before reaching Vmax is detected in plurality, a minimum point among the detected points (however, points in initial noise are excluded) is selected as $p_k$. As described earlier, $p_k$ and $q_k$ may be data based on the number of measurement points or data based on time.

A value of the variable $X_k$ can be optionally set. Preferably, $X_k$ is larger than 0 and smaller than Vmax. $X_k$ can be set based on Vmax. For example, $X_k$ is specified by Vmax $\times S_k$(%), where $S_k$ need only be larger than 0 and smaller than 100 and, preferably, $S_k$ can be set within a range from 0.5 to 99. When setting n-number of $X_k$ ($X_1, ..., X_n$), n-number of $S_k$ ($S_1, ..., S_n$) are set (where k and n are as described above, and each $S_k$ is larger than 0 and smaller than 100 and preferably ranges from 0.5 to 99). While the number of the variable $X_k$ to be set is not particularly limited, the number preferably ranges from 5 to 50 and more preferably ranges from 10 to 30 or, in other words, k is an integer preferably ranging from 5 to 50 and more preferably ranging from 10 to 30.

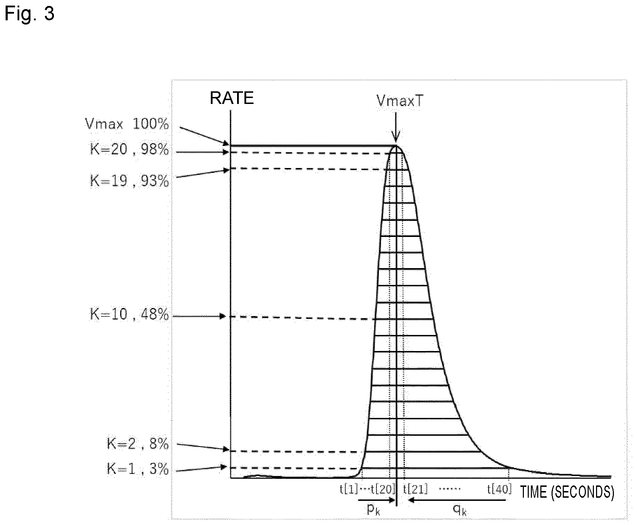

$X_k$, $p_k$, and $q_k$ will now be described with reference to Figure 3. In Figure 3, the first derivative V of a coagulation reaction curve is plotted against time. A peak top of V is a maximum value Vmax (100%) and a time satisfying V = Vmax is represented by VmaxT. 20 $X_k$ (where k denotes integers from 1 to 20) are set, and each $X_k$ is specified as a value ranging from Vmax $\times$ 3% (k = 1) to Vmax $\times$ 98% (k = 20). A line indicating each $X_k$ is drawn under a curve of V. With respect to each $X_k$, there are two time points where V assumes $X_k$ (intersections of the line of $X_k$ and V) of which one exists before VmaxT and the other exists after VmaxT. As a result, 40 points of time where V = $X_k$ is satisfied are detected with respect to the 20 $X_k$. Among these time points, 20 time points (from t[1] to t[20]) before VmaxT correspond to $p_k$ and 20 time points (from t[40] to t[21]) after VmaxT correspond to $q_k$.

$p_k$ and $q_k$ described above can be acquired as second data. Alternatively, the second data may include $p_k$ and $q_k$ or may not include $p_k$ and $q_k$ and include a parameter calculated from $p_k$ or $q_k$. Examples of the parameter which can be included in the second data include statistics of $p_k$ or $q_k$. Examples of the statistics of $p_k$ or $q_k$ include an average value (Ave), a standard deviation (SD), and a coefficient of variation (CV) of $p_k$ ($p_1, ..., p_n$) or $q_k$ ($q_1, ..., q_n$). In the present specification, the Ave, the SD, and the CV of the point $p_k$ which exists before the point where V reaches Vmax may be respectively referred to as a pre-Ave, a pre-SD, and a pre-CV and, in a similar manner, the Ave, the SD, and the CV of the point $q_k$ which exists after the point where V reaches Vmax may be respectively referred to as a post-Ave, a post-SD, and a post-CV. Further examples of the statistics of $p_k$ or $q_k$ include a ratio between a difference between the pre-Ave and the post-Ave and an average value of all $p_k$ and $q_k$ ([(post-Ave - pre-Ave)/(average value of $p_k$ and $q_k$)], also referred to as a pre-post average difference) and a ratio between the pre-SD and the post-SD ([pre-SD/post-SD], also referred to as a pre-post SD ratio).

Further examples of the parameter which can be included in the second data include a midpoint $M_k$ of $p_k$ and $q_k$ and a peak width $W_k$ representing a width from $p_k$ to $q_k$ obtained by the equations provided below, and a coefficient of variation (CV) of the midpoint $M_k$. In the following equations, k represents a series of integers from 1 to n, where n denotes an integer equal to or larger than 2. Therefore, n-number of $M_k$ ($M_1$, ..., $M_n$) and n-number of $W_k$ ($W_1$, ..., $W_n$) can be calculated. The CV of the midpoint $M_k$ ($M_1$, ..., $M_n$) reflects a degree of distortion of a peak shape of V and is also referred to as a distortion index in the present specification. In other words, since the more distorted (the larger the asymmetry of) the peak shape of V, the larger a change in $M_k$, the larger the CV of $M_k$ or, in other words, the larger the distortion index.

$$M_k = (p_k + q_k)/2$$

$$W_k = q_k - p_k$$

Further examples of the parameter which can be included in the second data include a peakedness index which reflects a peakedness of the peak shape of V. For example, the peakedness index is represented by a ratio between sums (or average values) of $W_k$ ($W_1$, ..., $W_n$) of an upper part and a lower part of a peak of V obtained by the following equation.

$$\text{peakedness index} = (\text{sum or average value of } W_k \text{ with respect to lower part of peak of V})/(\text{sum or average value of } W_k \text{ with respect to upper part of peak of V})$$

For example, when 20 $X_k$ are set at regular intervals as shown in Figure 3, $W_1$ to $W_{10}$ represent $W_k$ with respect to a lower half of the peak of V while $W_{11}$ to $W_{20}$ represent $W_k$ with respect to an upper half of the peak of V. In this case, the peakedness index is a value obtained by dividing a sum (or an average value) of $W_1$ to $W_{10}$ by a sum (or an average value) of $W_{11}$ to $W_{20}$. In other words, since the more peaked the peak of V, the larger the difference in peak widths between the upper part and the lower part of the peak of V, the larger the peakedness index.

In an embodiment, the second data acquired by the method according to the present invention may include only at least $p_k$ and $q_k$ and, preferably, includes $p_k$ and $q_k$ and at least one selected from the group consisting of the parameters calculated from $p_k$ or $q_k$ described above. In an example, the second data includes $p_k$ and $q_k$ and at least one selected from the group consisting of the pre-Ave, the post-Ave, the pre-SD, the post-SD, the pre-CV, the post-CV, the pre-post average difference, the pre-post SD ratio, $M_k$, $W_k$, the distortion index, and the peakedness index. In a preferable example, the second data includes $p_k$, $q_k$, the pre-CV, and the post-CV. In another preferable example, the second data includes $p_k$, $q_k$, the pre-post average difference, and the pre-post SD ratio. In another preferable example, the second data includes $p_k$, $q_k$, $M_k$, and $W_k$. In another preferable example, the second data includes $p_k$, $q_k$, the distortion index, and the peakedness index. In a further preferable example, the second data includes $p_k$, $q_k$, the pre-CV, the post-CV, the pre-post average difference, the pre-post SD ratio, the distortion index, and the peakedness index.

In an embodiment, the second data acquired by the method according to the present invention includes at least one selected from the group at least consisting of parameters calculated from $p_k$ or $q_k$ described above. In an example, the second data includes at least one selected from the group consisting of the pre-Ave, the post-Ave, the pre-SD, the post-SD, the pre-CV, the post-CV, the pre-post average difference, the pre-post SD ratio, $M_k$, $W_k$, the distortion index, and the peakedness index. In a preferable example, the second data includes the pre-CV and the post-CV. In another preferable example, the second data includes the pre-post average difference and the pre-post SD ratio. In another preferable example, the second data includes $M_k$ and $W_k$. In another preferable example, the second data includes the distortion index and the peakedness index. In a further preferable example, the second data includes the pre-CV, the post-CV, the pre-post average difference, the pre-post SD ratio, the distortion index, and the peakedness index.

In addition to $p_k$ and $q_k$ or a parameter calculated from $p_k$ or $q_k$, V itself, the maximum value Vmax of V, or VmaxT which represents a time or the number of measurement points where V = Vmax is satisfied may be further included in the second data.

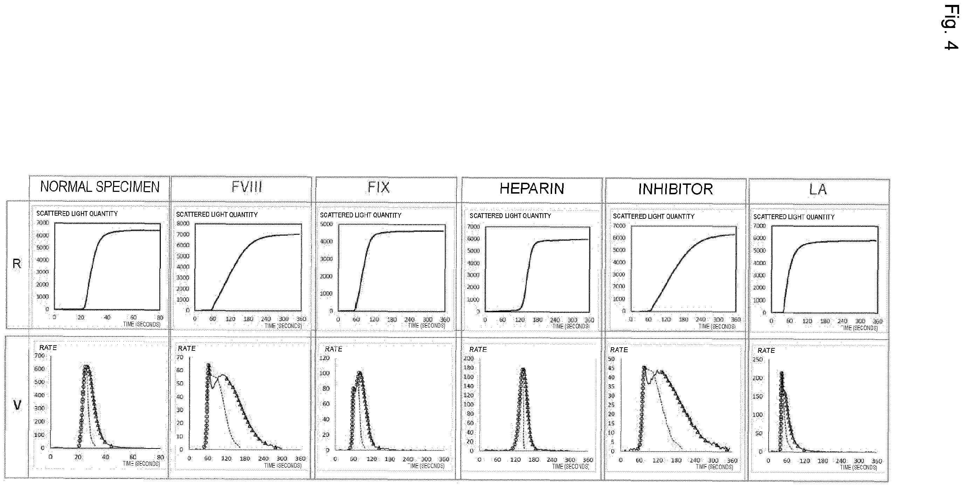

A shape of a coagulation reaction curve of a specimen tends to differ dependent on blood coagulation characteristics (in other words, a blood coagulation abnormality factor) of the specimen, and the tendency is reflected on $p_k$, $q_k$, and a parameter calculated from $p_k$ or $q_k$ described above. As shown in an upper part of Figure 4, an abnormal specimen having a coagulation abnormality factor typically has a coagulation reaction curve R of which shape differs from that of a normal specimen and has a prolonged coagulation time. In addition, as shown in a lower part of Figure 4, V of an abnormal specimen tends to have a smaller peak as compared to a normal specimen and, dependent on a prolongation factor, tends to exhibit a different shape such as a wide shape, an asymmetric shape, or a bimodal shape. On each V in the lower part of Figure 4, points $(p_k, X_k)$ (circle) and $(q_k, X_k)$ (triangle) where $V = X_k$ (k = 1 to 20) is satisfied are marked before and after VmaxT and, further, lines connecting $(M_k, X_k)$ are indicated by dotted lines.

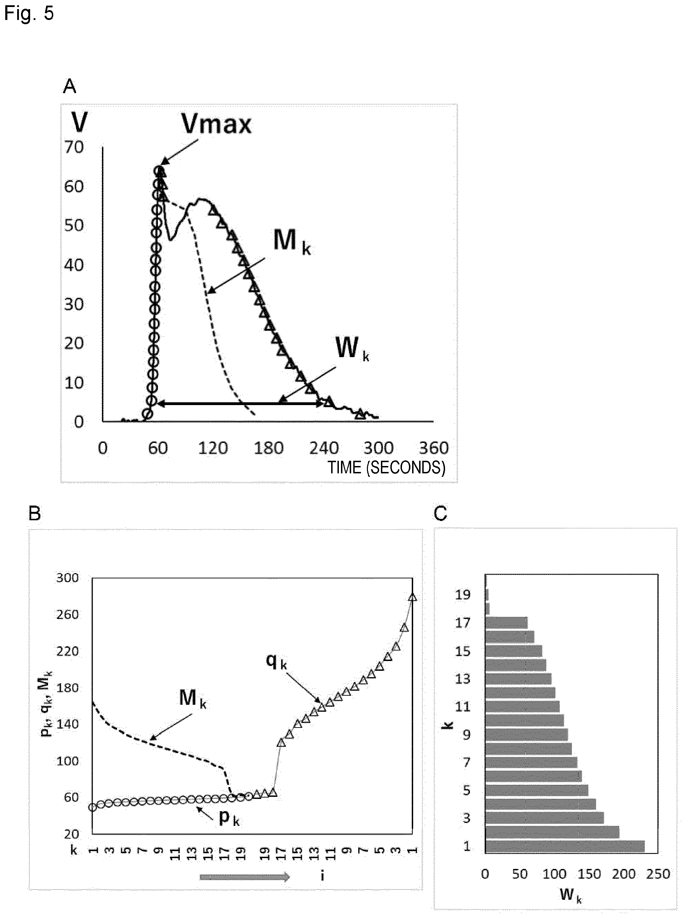

As a more detailed example, a parameter of a coagulation factor VIII (FVIII)-deficient specimen will be described with reference to Figure 5. Figure 5A shows V of an FVIII-deficient specimen on which points $(p_k, X_k)$ are marked by circles and points $(q_k, X_k)$ are marked by triangles, and lines connecting $(M_k, X_k)$ are indicated by dotted lines. A width from $p_k$ to $q_k$ is $W_k$. Figure 5B is a chart in which $p_k$, $q_k$, and $M_k$ shown in Figure 5A are plotted against time. Figure 5C is a chart representing a change in $W_k$ obtained from $p_k$ and $q_k$ shown in Figure 5A due to k. The FVIII-deficient specimen exhibits a prolonged coagulation time (coagulation time: 120 seconds) and, as shown in Figure 5A, V of the specimen is bimodal and once V temporarily decreases after abruptly rising until Vmax, V exhibits a relatively gradual second increase and decrease. Therefore, in the specimen, a change in $q_k$ is large as compared to a change in $p_k$ and a change in $M_k$ is also large (Figure 5B). $W_k$ of the specimen is extremely small in a portion of the first sharp peak (where k ranges from 18 to 20) but, in other portions, reflects the gradual peak of the latter half and becomes wider (Figure 5C). Therefore, in the specimen, while the pre-CV is small, the post-CV and the distortion index are relatively large and the peakedness index is also large.

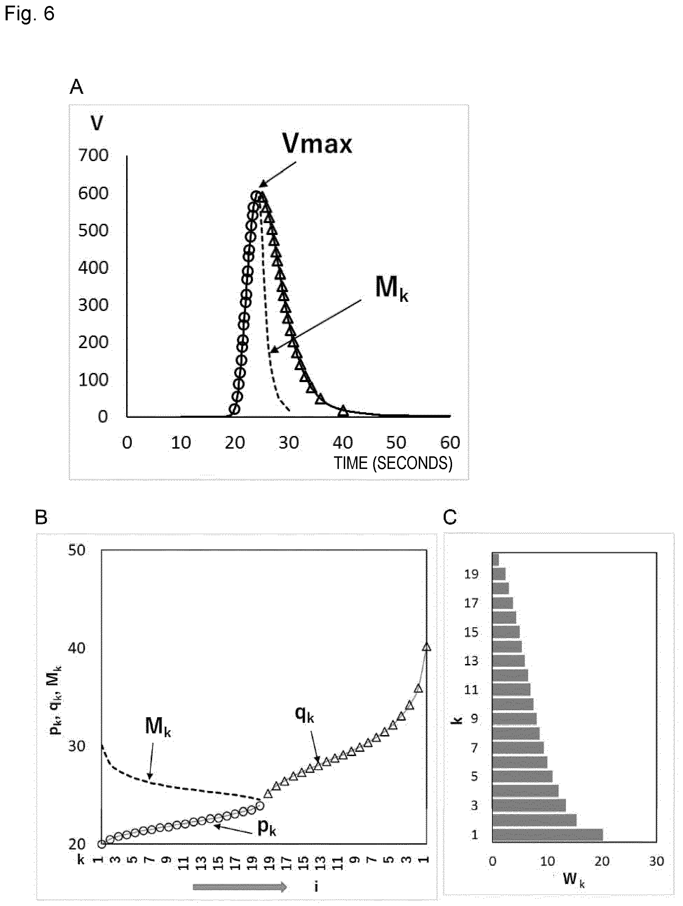

Figure 6A shows V of a normal specimen. Meanings of circles, triangles, and dotted lines in the diagram are the same as in Figure 5A. Figure 6B is a chart in which $p_k$, $q_k$, and $M_k$ shown in Figure 6A are plotted against time in a similar manner to Figure 5B. Figure 6C is a chart representing a change in $W_k$ obtained from $p_k$ and $q_k$ shown in Figure 6A due to k. The specimen is not subject to prolongation of a coagulation time and exhibits a normal coagulation time (coagulation time: 26 seconds) and V of the specimen has a unimodal peak shape in which Vmax is larger than that of the FVIII-deficient specimen shown in Figure 5A. Therefore, in the specimen, changes in $p_k$, $q_k$, and $M_k$ are smaller than in the FVIII-deficient specimen shown in Figure 5A (Figure 6B). In addition, $W_k$ of the specimen is smaller than that of the FVIII-deficient specimen shown in Figure 5A as a whole and a change thereof is also smaller (Figure 6C). Therefore, in the normal specimen, the distortion index and the peakedness index are smaller as compared to the FVIII-deficient specimen.

As described above, $p_k$, $q_k$, and parameters calculated from $p_k$ or $q_k$ described above which can be included in the second data reflects a shape of V of a specimen and, by extension, blood coagulation characteristics of the specimen. Therefore, a coagulation abnormality factor of the specimen can be estimated based on the second data.

5. Estimation of coagulation abnormality factor

In the method according to the present invention, a coagulation abnormality factor of an abnormal specimen of which a coagulation time has been prolonged (in other words, a prolongation factor of a coagulation time) can be estimated based on the second data. In addition, in the method according to the present invention, even with respect to a specimen in which prolongation of a coagulation time is not observed, a coagulation abnormality factor which potentially exists in the specimen can be estimated based on the second data in a similar manner. Furthermore, in the method according to the present invention, a presence or absence of a coagulation abnormality factor of a specimen can be estimated based on the second data. In the following present specification, a coagulation abnormality factor of an abnormal specimen of which a coagulation time has been prolonged (in other words, a prolongation factor of a coagulation time) and a coagulation abnormality factor which potentially exists in a specimen in which prolongation of a coagulation time is not observed will be collectively referred to as a coagulation abnormality factor (or simply an abnormality factor). In addition, in the following present specification, an estimation of a type of a coagulation abnormality factor and an estimation of a presence or absence of a coagulation abnormality factor will be collectively referred to as an estimation of a coagulation abnormality factor.

Examples of types of a coagulation abnormality factor which can be estimated by the method according to the present invention include a coagulation factor deficiency, lupus anticoagulant (LA)-positive, a coagulation factor inhibitor (inhibitor), and heparin-positive (to be precise, a heparin-containing specimen). Examples of a coagulation factor deficiency include a coagulation factor V (FV) deficiency, a coagulation factor VIII (FVIII) deficiency, a coagulation factor IX (FIX) deficiency, a coagulation factor X (FX) deficiency, a coagulation factor XI (FXI) deficiency, and a coagulation factor XII (FXII) deficiency. Examples of inhibitors include an FVIII inhibitor. Preferably, the type of a coagulation abnormality factor which can be estimated by the method according to the present invention is selected from the group consisting of a coagulation factor deficiency, LA-positive, an inhibitor, and heparin-positive and, more preferably, selected from the group consisting of an FVIII deficiency, an FIX deficiency, LA-positive, an FVIII inhibitor, and heparin-positive.

In an embodiment of the method according to the present invention, an abnormality factor of a subject specimen can be estimated based on relative values of the pre-CV and the post-CV of the specimen with respect to the pre-CV and the post-CV based on a normal specimen group. The pre-CV and the post-CV based on the normal specimen group are, for example, average values of the pre-CV and the post-CV of a plurality of normal specimens and are also referred to as a reference_pre-CV and a reference post-CV in the present specification. Data with respect to the normal specimens may be prepared in advance before measurement of the subject specimen or may be acquired by measuring the normal specimens together with the subject specimen.

In the embodiment, a value obtained by subtracting the reference_pre-CV from the pre-CV of the subject specimen is a relative_pre-CV of the subject specimen, and a value obtained by subtracting the reference_post-CV from the post-CV of the subject specimen is a relative_post-CV of the subject specimen.

In another embodiment, the relative_pre-CV of the subject specimen is represented by (pre-CV of subject specimen/reference_pre-CV) - 1, and the relative_post-CV of the subject specimen is represented by (post-CV of subject specimen/reference_post-CV) - 1.

An abnormality factor of the subject specimen can be estimated from the relative_pre-CV and the relative_post-CV of the subject specimen. For example, with respect to each of a normal specimen and various abnormal specimen types (for example, an FVIII deficiency, an FIX deficiency, LA-positive, an FVIII inhibitor, and heparin-positive), by determining reference values (or ranges) of the relative_pre-CV and the relative_post-CV in advance and studying either the reference values of any specimen type which the relative_pre-CV and the relative_post-CV of the subject specimen most closely resemble or the reference ranges of any specimen type which includes the relative_pre-CV and the relative_post-CV of the subject specimen, an abnormality factor of the subject specimen can be estimated.

In an embodiment of the method according to the present invention, an abnormality factor of a subject specimen can be estimated based on relative values of the distortion index and the peakedness index of the specimen with respect to the distortion index and the peakedness index based on a normal specimen group. The distortion index and the peakedness index based on the normal specimen group are, for example, average values of the distortion index and the peakedness index of a plurality of normal specimens and are also referred to as a reference_distortion index and a reference_peakedness index in the present specification. Data with respect to the normal specimens may be prepared in advance before measurement of the subject specimen or may be acquired by measuring the normal specimens together with the subject specimen.

In the embodiment, a value obtained by subtracting the reference_distortion index from the distortion index of the subject specimen is a relative_distortion index of the subject specimen, and a value obtained by subtracting the reference_peakedness index from the peakedness index of the subject specimen is a relative_peakedness index of the subject specimen.

In another embodiment, the relative_distortion index of the subject specimen is represented by (distortion index of subject specimen/reference_distortion index) - 1, and the relative_peakedness index of the subject specimen is represented by (peakedness index of subject specimen/reference_peakedness index) - 1.

An abnormality factor of the subject specimen can be estimated from the relative_distortion index and the relative_peakedness index of the subject specimen. For example, in a similar manner to the relative_pre-CV and the relative_post-CV described above, by studying either the reference values of any specimen type which the relative_distortion index and the relative_peakedness index of the subject specimen most closely resemble or the reference ranges of any specimen type which includes the relative_distortion index and the relative_peakedness index of the subject specimen, an abnormality factor of the subject specimen can be estimated.

In an embodiment of the method according to the present invention, an abnormality factor of a subject specimen can be estimated based on relative values of the pre-post average difference and the pre-post SD ratio of the specimen with respect to the pre-post average difference and the pre-post SD ratio based on a normal specimen group. The pre-post average difference and the pre-post SD ratio based on the normal specimen group are, for example, average values of the pre-post average difference and the pre-post SD ratio of a plurality of normal specimens and are also referred to as a reference_pre-post average difference and a reference_pre-post SD ratio in the present specification. Data with respect to the normal specimens may be prepared in advance before measurement of the subject specimen or may be acquired by measuring the normal specimens together with the subject specimen.

In the embodiment, a value obtained by subtracting the reference_pre-post average difference from the pre-post average difference of the subject specimen is a relative_pre-post average difference of the subject specimen, and a value obtained by subtracting the reference_pre-post SD ratio from the pre-post SD ratio of the subject specimen is a relative_pre-post SD ratio of the subject specimen.

In another embodiment, the relative_pre-post average difference of the subject specimen is represented by (pre-post average difference of subject specimen/reference_pre-post average difference) - 1, and the relative_pre-post SD ratio of the subject specimen is represented by (pre-post SD ratio of subject specimen/reference_pre-post SD ratio) - 1.

An abnormality factor of the subject specimen can be estimated from the relative_pre-post average difference and the relative_pre-post SD ratio of the subject specimen. For example, in a similar manner to the relative_pre-CV and the relative_post-CV described above, by studying either the reference values of any specimen type which the relative_pre-post average difference and the relative_pre-post SD ratio of the subject specimen most closely resemble or the reference ranges of any specimen type which includes the relative_pre-post average difference and the relative_pre-post SD ratio of the subject specimen, an abnormality factor of the subject specimen can be estimated.

In another embodiment of the method according to the present invention, an abnormality factor of a subject specimen can be estimated based on a standard deviation interval (SDI) of a parameter calculated from $p_k$ or $q_k$ described above with respect to the specimen. The SDI of an objective parameter with respect to the subject specimen is calculated by the following equation.

SDI of objective parameter with respect to the subject specimen = $(\alpha - \beta) \div \gamma$, wherein
$\alpha$: value of objective parameter from subject specimen
$\beta$: reference value of objective parameter based on data of normal specimen group
$\gamma$: reference deviation of objective parameter based on data of normal specimen group

In the equation provided above, the reference value ($\beta$) and the reference deviation ($\gamma$) based on data of a normal specimen group can be determined in advance.

For example, an average value and a standard deviation of the objective parameter calculated from a given normal specimen group can be respectively applied to the reference value ($\beta$) and the reference deviation ($\gamma$). Alternatively, the reference value ($\beta$) and the reference deviation ($\gamma$) can be determined by the following procedure: calculate an average value of an objective parameter from an arbitrarily determined first normal specimen group and exclude a specimen having an objective parameter which deviates from the average value (for example, deviates by more than $\pm 3$ SD) from the first normal specimen group. Next, the same procedure is repeated using a second normal specimen group made up of remaining specimens. The procedures described above are repeated until, finally, specimens having an objective parameter which deviates from the average value are no longer detected, and an average value and a standard deviation of an objective parameter from a normal specimen group made up of last remaining specimens are adopted as the reference value ($\beta$) and the reference deviation ($\gamma$).

The SDI represents a bias of the parameter value ($\alpha$) from the subject specimen with respect to the reference value ($\beta$) based on a normal specimen. For example, when the SDI of a parameter $\alpha1$ in the subject specimen is 3 and the SDI of a parameter $\alpha2$ is -4, the value of the parameter $\alpha1$ is higher than the reference value ($\beta$) by 3SD and the value of the parameter $\alpha2$ is lower than the reference value ($\beta$) by 4SD in the subject specimen. Converting a parameter value into an SDI enables a difference between a subject specimen and a normal specimen to be assessed at a same scale (based on a relative value with respect to an SD) in all parameters. Furthermore, since obtaining an SDI enables different parameters to be compared with each other, a parameter more suitable for estimating an abnormality factor (a parameter which enables estimation of an abnormality factor to be performed with higher accuracy) can be selected.

An abnormality factor of the subject specimen can be estimated from the SDI of the parameter of the subject specimen. For example, with respect to each of a normal specimen and various abnormal specimen types (for example, an FVIII deficiency, an FIX deficiency, LA-positive, an FVIII inhibitor, and heparin-positive), a standard value (or range) of the SDI of various parameters is to be determined in advance. By studying either the reference value of any specimen type which the SDI of a same parameter of the subject specimen most closely resembles or the reference range of any specimen type which includes the SDI of the same parameter of the subject specimen, an abnormality factor of the subject specimen can be estimated.

In a more detailed example, an abnormality factor of the subject specimen can be estimated using a two-dimensional plot of a parameter or an SDI thereof. In this case, a two-dimensional plot using any two parameters selected from the parameters described above or any two SDIs selected from SDIs of the parameters described above is used. In a preferable example, the parameters used in the two-dimensional plot are any two selected from the group consisting of the pre-CV, the post-CV, the distortion index, the peakedness index, the pre-post average difference, and the pre-post SD ratio. In another preferable example, the parameters used in the two-dimensional plot are any two selected from the group consisting of the relative_pre-CV, the relative_post-CV, the relative_distortion index, the relative_peakedness index, the relative_pre-post average difference, and the relative_pre-post SD ratio. In another preferable example, the parameters used in the two-dimensional plot are any two selected from the group consisting of an SDI of the pre-CV, an SDI of the post-CV, an SDI of the distortion index, an SDI of the peakedness index, an SDI of the pre-post average difference, and an SDI of the pre-post SD ratio.

A procedure of estimating an abnormality factor with a two-dimensional plot will be described using the pre-CV and the post-CV as an example. The pre-CV and the post-CV of a subject specimen are plotted as a single point (pre-CV, post-CV) or (post-CV, pre-CV) on a two-dimensional plane of the pre-CV and the post-CV (refer to Figure 9A). On the other hand, the reference_pre-CV and the reference_post-CV are also plotted as a single point on the two-dimensional plane and the point is specified as a reference point. By shifting the plot so that the reference point overlaps with an origin of the two-dimensional plane, the relative_pre-CV and the relative_post-CV of the subject specimen are plotted on the two-dimensional plane. Alternatively, the relative_pre-CV and the relative_post-CV obtained as described above may be two-dimensionally plotted (refer to Figure 9B).

An abnormality factor of the subject specimen can be estimated based on a position of the plot of the relative_pre-CV and the relative_post-CV on the two-dimensional plane. For example, with respect to each of a normal specimen and various abnormal specimen types (for example, an FVIII deficiency, an FIX deficiency, LA-positive, an FVIII inhibitor, and heparin-positive), by determining a standard distribution area of the plot of the relative_pre-CV and the relative_post-CV on the two-dimensional plane in advance and studying a standard distribution area of any specimen type which includes the plot of the subject specimen, an abnormality factor of the subject specimen can be estimated.

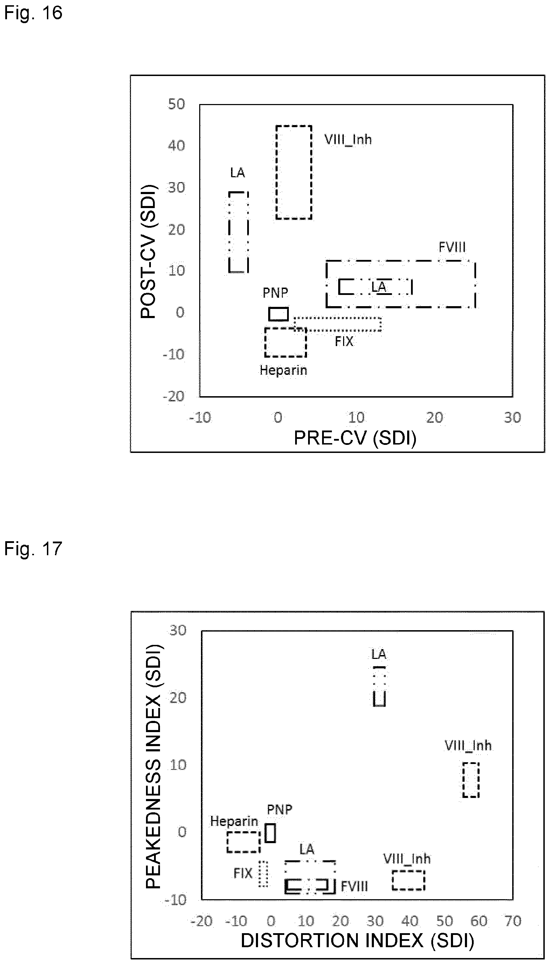

When using an SDI, the SDI of the pre-CV and the SDI of the post-CV of the subject specimen are plotted as a single point (SDI of pre-CV, SDI of post-CV) or (SDI of post-CV, SDI of pre-CV) on a two-dimensional plane (refer to Figure 9C). A presence or absence of an abnormality or an abnormality factor of the subject specimen can be estimated based on a position of the plot on the two-dimensional plane. For example, with respect to each of a normal specimen and various abnormal specimen types (for example, an FVIII deficiency, an FIX deficiency, LA-positive, an FVIII inhibitor, and heparin-positive), by determining a standard distribution area of the plot of the SDI of the pre-CV and the SDI of the post-CV on the two-dimensional plane in advance (refer to Figure 16) and studying a standard distribution area of any specimen type which includes the plot of the subject specimen, an abnormality factor of the subject specimen can be estimated.

As an example, the following estimation can be performed with reference to Table 1-2, Figure 9-C, and Figure 16 to be described later:

When the SDI of the pre-CV of the subject specimen exceeds ±3 or the SDI of the post-CV of the subject specimen exceeds ±3, it is estimated that the subject specimen has a coagulation abnormality factor;

When the SDI of the pre-CV of the subject specimen is 6.2 or more and 25.2 or less and the SDI of the post-CV of the subject specimen is 1.3 or more and 12.5 or less, it is estimated that an abnormality factor of the subject specimen is possibly an FVIII deficiency;

In the subject specimen having been estimated that there is a possibility of an FVIII deficiency, when the SDI of the pre-CV is 7.8 or more and 17.1 or less and the SDI of the post-CV is 4.6 or more and 8.0 or less, it is estimated that an abnormality factor of the subject specimen is possibly LA-positive;

When the SDI of the pre-CV of the subject specimen is 2.1 or more and 13.1 or less and the SDI of the post-CV of the subject specimen is -4.2 or more and -1.2 or less, it is estimated that an abnormality factor of the subject specimen is an FIX deficiency;

When the SDI of the pre-CV of the subject specimen is -0.2 or more and 4.3 or less and the SDI of the post-CV of the subject specimen is 22.6 or more and 44.7 or less, it is estimated that an abnormality factor of the subject specimen is an FVIII inhibitor;

When the SDI of the pre-CV of the subject specimen is -6.2 or more and -3.9 or less and the SDI of the post-CV of the subject specimen is 9.8 or more and 28.9 or less, it is estimated that an abnormality factor of the subject specimen is LA-positive; and

When the SDI of the pre-CV of the subject specimen is -1.6 or more and 3.6 or less and the SDI of the post-CV of the subject specimen is -10.4 or more and -3.7 or less, it is estimated that an abnormality factor of the subject specimen is heparin-positive.

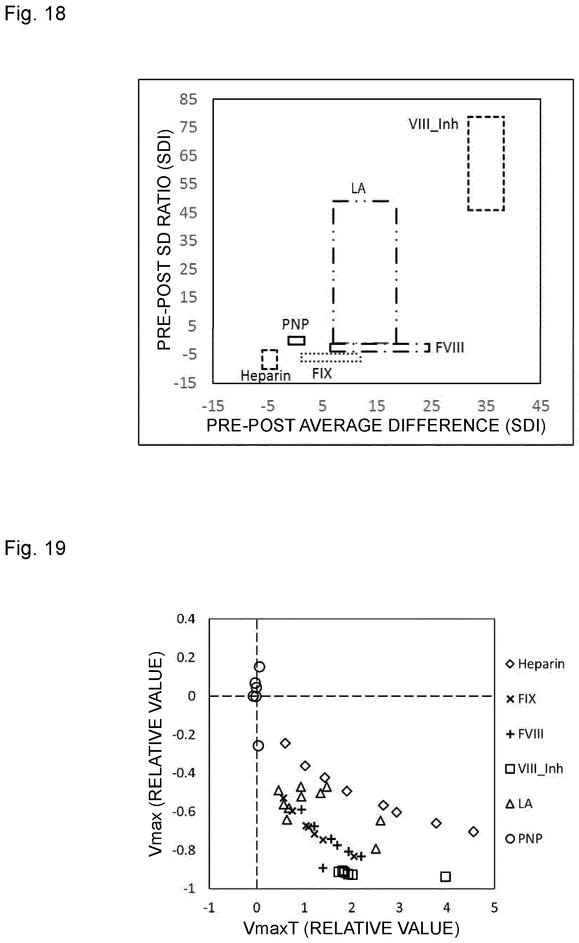

Similar estimations may be performed with respect to the distortion index and the peakedness index and with respect to the pre-post average difference and the pre-post SD ratio. In other words, procedures similar to those used in the case of the pre-CV and the post-CV described above can be used with respect to a plot by the relative_distortion index and the relative_peakedness index, a plot by the relative_pre-post average difference and the relative_pre-post SD ratio, a plot by the SDI of the distortion index and the SDI of the peakedness index, and a plot by the SDI of the pre-post average difference and the SDI of the pre-post SD ratio. For example, with respect to each of a normal specimen and various abnormal specimen types, by determining a standard distribution area of the plot of the SDIs of the distortion index and the peakedness index or the plot of the SDIs of the pre-post average difference and the pre-post SD ratio on the two-dimensional plane in advance (refer to Figures 17 and 18) and studying a standard distribution area of any specimen type which includes the plot of the subject specimen, an abnormality factor of the subject specimen can be estimated.

When two or more abnormality factors are estimated from a subject specimen in the estimation described above, an estimated abnormality factor of the subject specimen can be narrowed down by performing an estimation with the procedures described above using a different parameter or performing another test (for example, a conventionally known prolongation factor differential test such as a cross-mixing test).

For example, when both an FVIII deficiency and LA-positive are estimated as an abnormality factor of the subject specimen in an estimation using a two-dimensional plot of the SDI of the pre-CV and the SDI of the post-CV as shown in Figure 16, whether an estimated abnormality factor of the subject specimen is an FVIII deficiency or LA-positive can be determined by performing an estimation using a two-dimensional plot of the SDI of the pre-post average difference and the SDI of the pre-post SD ratio as shown in Figure 18. Alternatively, whether the estimated abnormality factor is an FVIII deficiency or LA-positive can be determined by performing an immediate reaction of a cross-mixing test of the subject specimen.

6. Coagulation abnormality factor estimation by machine learning model

In another embodiment of the method according to the present invention, a coagulation abnormality factor of a specimen can be estimated in accordance with a coagulation abnormality factor estimation model (machine learning model) constructed by machine learning. As supervised data for machine learning, data of a blood coagulation reaction from a supervised specimen group and data with respect to a presence or absence of a coagulation abnormality or a type of an abnormality factor are used. For example, a machine learning model for estimating a coagulation abnormality factor of a subject specimen is constructed by machine learning which uses a feature amount representing a blood coagulation reaction of each specimen in the supervised specimen group as an explanatory variable and which uses data with respect to a presence or absence of a coagulation abnormality or a type of an abnormality factor of each specimen in the supervised specimen group as a target variable.

As the supervised specimen group, a blood specimen group of which a blood coagulation reaction and a presence or absence of a coagulation abnormality or a type of an abnormality factor are known is used. In an embodiment, the supervised specimen group includes a blood specimen (normal specimen) without a coagulation abnormality and a blood specimen (abnormal specimen) with a coagulation abnormality. In an embodiment, the supervised specimen group includes abnormal specimens respectively having different coagulation abnormality factors (preferably, a coagulation factor deficiency, LA-positive, an inhibitor, heparin-positive, and the like). Preferably, the supervised specimen group includes a normal specimen and abnormal specimens respectively having different coagulation abnormality factors (preferably, a coagulation factor deficiency, LA-positive, an inhibitor, heparin-positive, and the like).

Examples of a feature amount with respect to a blood coagulation reaction used in the explanatory variable include at least one selected from the group consisting of parameters acquired in the second data acquisition step described above.

In a preferable example, the explanatory variable is at least one selected from the group consisting of the pre-CV, the post-CV, the pre-post average difference, the pre-post SD ratio, the distortion index, and the peakedness index and, more preferably, at least one selected from the group consisting of a combination of the pre-CV and the post-CV, a combination of the pre-post average difference and the pre-post SD ratio, and a combination of the distortion index and the peakedness index. Alternatively, all of the pre-CV, the post-CV, the pre-post average difference, the pre-post SD ratio, the distortion index, and the peakedness index may be used as the explanatory variable.

In another preferable example, the explanatory variable is at least one selected from the group consisting of a relative_pre-CV, a relative_post-CV, a relative_pre-post average difference, a relative_pre-post SD ratio, a relative_distortion index, and a relative_peakedness index and, more preferably, at least one selected from the group consisting of a combination of the relative_pre-CV and the relative_post-CV, a combination of the relative_pre-post average difference and the relative_pre-post SD ratio, and a combination of the relative_distortion index and the relative_peakedness index. Alternatively, all of the relative_pre-CV, the relative_post-CV, the relative_pre-post average difference, the relative_pre-post SD ratio, the relative_distortion index, and the relative_peakedness index may be used as the explanatory variable.

In another preferable example, the explanatory variable is at least one selected from the group consisting of an SDI of the pre-CV, an SDI of the post-CV, an SDI of the pre-post average difference, an SDI of the pre-post SD ratio, an SDI of the distortion index, and an SDI of the peakedness index and, more preferably, at least one selected from the group consisting of a combination of the SDI of the pre-CV and the SDI of the post-CV, a combination of the SDI of the pre-post average difference and the SDI of the pre-post SD ratio, and a combination of the SDI of the distortion index and the SDI of the peakedness index. Alternatively, all of the SDI of the pre-CV, the SDI of the post-CV, the SDI of the pre-post average difference, the SDI of the pre-post SD ratio, the SDI of the distortion index, and the SDI of the peakedness index may be used as the explanatory variable.

Together with the above, other parameters such as at least one selected from the group consisting of $p_k$, $q_k$, the pre-Ave, the post-Ave, the pre-SD, the post-SD, $M_k$, $W_k$, Vmax, VmaxT, and the like may be added to the explanatory variable.

Examples of data of a presence or absence of a coagulation abnormality or data of a type of an abnormality factor include data representing a presence or absence of a coagulation abnormality or data representing a type of an abnormality factor (for example, a coagulation factor deficiency, LA-positive, an inhibitor, or heparin-positive).

Examples of a machine learning algorithm used to construct the machine learning model include known machine learning algorithms such as a support vector machine (SVM), a neural network (NN), a decision tree, a random forest, and k-nearest neighbors.

Data for verification is input to the constructed model to calculate an estimation result of a coagulation abnormality factor. A model in which the estimation result best matches an actual result such as a model in which the estimation result has a highest accuracy rate with respect to the actual result or a model with a smallest error between the estimation result and the actual result can be selected as an optimal model.

The constructed machine learning model outputs, from a feature amount with respect to a blood coagulation reaction with respect to a subject specimen (in other words, data corresponding to the explanatory variable described earlier), an estimation result of a presence or absence of a coagulation abnormality or a type of an abnormality factor (for example, a coagulation factor deficiency, LA, an inhibitor, or heparin-positive) of the subject specimen.

In an embodiment, the machine learning model is a model for estimating the presence or absence of a coagulation abnormality and, by inputting a feature amount with respect to a blood coagulation reaction of a subject specimen, a presence or absence of a coagulation abnormality of the subject specimen (for example, whether the subject specimen is normal or has a coagulation abnormality) is estimated.

In another embodiment, the machine learning model is a model for estimating a coagulation abnormality factor and, by inputting a feature amount with respect to a blood coagulation reaction of a subject specimen, a type of a coagulation abnormality factor of the subject specimen (for example, a coagulation factor deficiency, LA-positive, an inhibitor, or heparin-positive) is estimated.

For example, the machine learning model is a model for estimating the presence or absence of a coagulation abnormality and a type of an abnormality factor and, by inputting a feature amount with respect to a blood coagulation reaction of a subject specimen, a presence or absence of a coagulation abnormality of the subject specimen and, when abnormal, a type of the abnormality factor is estimated.

7. Application to other coagulation reaction measurement methods

A blood coagulation time measurement method according to the present invention has been described above using a coagulation reaction measurement based on a scattered light quantity as an example. However, a person skilled in the art should be able to apply the method according to the present invention to blood coagulation time measurement methods using other coagulation reaction measurement methods (for example, a blood coagulation reaction measurement method based on transmittance, absorbance, viscosity, or the like) and, therefore, such an application is included in a scope of the present invention. For example, a reaction R(i) obtained from a coagulation reaction curve with an inverse sigmoidal shape based on scattered light quantity has reverse polarity with respect to a coagulation reaction curve based on the scattered light quantity described above. In such a case, it should be apparent to a person skilled in the art that signs of R(i) and V(i) in steps 1 to 4 described above are to be reversed, a minimum value Vmin of V is to be determined instead of the maximum value Vmax, $p_k$ and $q_k$ are respectively determined as points where V(i) assumes $X_k$ before and after reaching Vmin, and the like.

8. Program and apparatus

The blood coagulation reaction analysis method according to the present invention described above can be automatically performed using a computer program. Therefore, an aspect of the present invention is a program for performing the blood coagulation reaction analysis method according to the present invention described above. In addition, the series of steps of the method according to the present invention described above can be automatically performed by an automated analyzer. Therefore, an aspect of the present invention is an apparatus for performing the blood coagulation reaction analysis method according to the present invention described above.

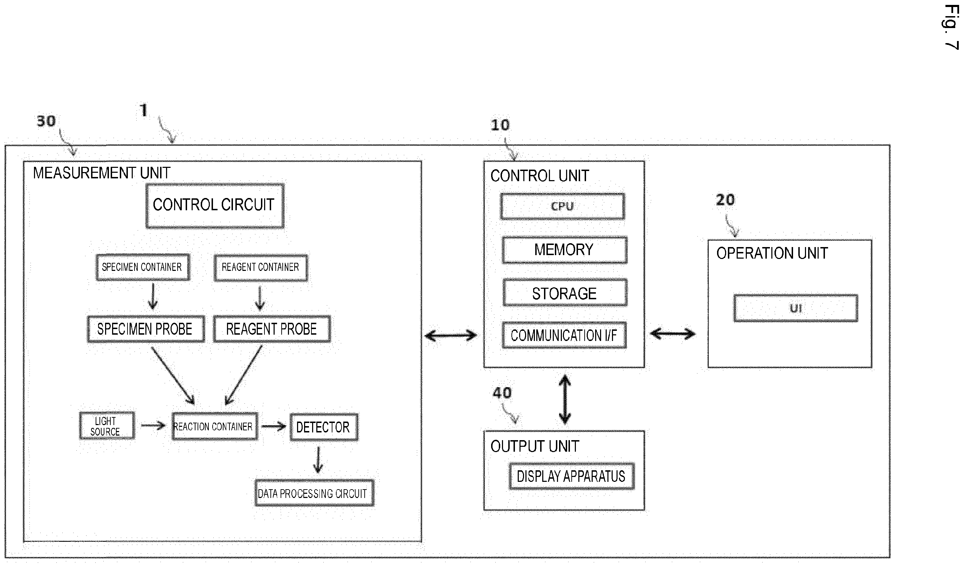

An embodiment of the apparatus according to the present invention will be described below. An embodiment of the apparatus according to the present invention is an automated analyzer 1 such as that shown in Figure 7. The automated analyzer 1 includes a control unit 10, an operation unit 20, a measurement unit 30, and an output unit 40.

The control unit 10 controls operations of the automated analyzer 1 as a whole. The control unit 10 can be constituted of, for example, a personal computer (PC). The control unit 10 includes a CPU, a memory, a storage, a communication interface (I/F), and the like and performs processing of a command from the operation unit 20, control of operations of the measurement unit 30, storage and data analysis of measured data received from the measurement unit 30, storage of an analysis result, control of output of measured data and/or an analysis result by the output unit 40, and the like. Furthermore, the control unit 10 may be connected to other devices such as an external medium and a host computer. In the control unit 10, a PC which controls operations of the measurement unit 30 may be the same as or may differ from a PC which performs analysis of measured data.

The operation unit 20 acquires input from an operator and transmits obtained input information to the control unit 10. For example, the operation unit 20 includes a user interface (UI) such as a keyboard or a touch panel. The output unit 40 outputs, under control by the control unit 10, measured data of the measurement unit 30, first data and/or second data based on the measured data and, when necessary, an analysis result such as a coagulation time or an estimation result of a coagulation abnormality factor or the like. For example, the output unit 40 includes a display apparatus such as a display.

The measurement unit 30 executes a series of operations for a blood coagulation test and acquires measured data of a coagulation reaction of a sample including a blood specimen. The measurement unit 30 includes various equipment and analysis modules necessary for a blood coagulation test such as a specimen container for storing a blood specimen, a reagent container for storing a test reagent, a reaction container for a reaction between the specimen and the reagent, a probe for dispensing the blood specimen and the reagent to the reaction container, a light source, a detector for detecting scattered light or transmitted light from the reagent in the reaction container, a data processing circuit which sends data from the detector to the control unit 10, and a control circuit which receives an instruction of the control unit 10 and which controls operations of the measurement unit 30.

The control unit 10 performs an analysis of a coagulation reaction of a specimen based on data measured by the measurement unit 30. The present analysis can include acquisition of the coagulation reaction curve R and the first derivative V, acquisition of the first data and the second data, calculation of a coagulation time using the first data, and estimation of a coagulation abnormality factor using the second data described above. Alternatively, the coagulation reaction curve R and the first derivative V may be created by the control unit 10 based on measured data from the measurement unit 30 or may be created by another device such as the measurement unit 30 and sent to the control unit 10. The control unit 10 may store reference values such as the reference_pre-CV and the reference_post-CV used in estimation of a coagulation abnormality factor, a reference value (β) and a reference deviation (γ) of various parameters, and the like or the control unit 10 may import, when performing an analysis, the reference values stored in an external device or on a network.

The estimation of a coagulation abnormality factor in the control unit 10 may be performed based on the machine learning model for coagulation abnormality factor estimation described above. In this case, preferably, the control unit 10 may store the machine learning model for coagulation abnormality factor estimation. The machine learning model may be constructed outside and sent to the control unit 10 to be stored or used or the machine learning model may be constructed and stored or used in the control unit 10.

The present analysis described above can be implemented by a program for performing the method according to the present invention. Therefore, the control unit 10 can include a program for performing the blood coagulation reaction analysis method according to the present invention.

An analysis result obtained by the control unit 10 is sent to the output unit 40 to be output. The output may take any form such as a display on a screen, a transmission to a host computer, a printout, or the like. Output information from the output unit can include waveform data of R or V, a coagulation time, an estimation result of a coagulation abnormality factor, information related to determination criteria of the coagulation time included in the first data, information related to Vmax, VmaxT, $p_k$, $q_k$, or a parameter calculated therefrom included in the second data, and a two-dimensional plot image of the parameter. Types of output information from the output unit can be controlled by the program according to the present invention.

In an embodiment of the apparatus according to the present invention, the measurement unit 30 continues measurement of a subject specimen until an end of a coagulation reaction and data is sequentially sent to the control unit 10. The control unit 10 sequentially continues calculations for acquiring the first data from the coagulation reaction curve R or the first derivative V and calculates a coagulation time in a timely manner. On the other hand, in parallel to the acquisition of the first data, the coagulation reaction curve R or the first derivative V is continuously acquired and R and V until the end of the coagulation reaction are acquired. Next, the control unit 10 acquires the second data and performs calculation for estimating a coagulation abnormality factor. An obtained analysis result is sent to the output unit to be output. For example, R and V are sequentially output until the end of the coagulation reaction in parallel with the measurement, a coagulation time is output midway through the measurement, and an estimation result of a coagulation abnormality factor is output after the end of the measurement.

Examples

While the present invention will be described below in greater detail by citing examples, the present invention is not limited by the following examples.

First example

1. Method

1.1) Specimen

- As six normal specimens (PNP), two specimens of citric acid-added pool plasma obtained from normal subjects (both N = 1), Calibrator N for Coagpia manufactured by SEKISUI MEDICAL CO., LTD. (N = 1), Coagtrol N manufactured by Sysmex Corporation (N = 1), Pooled Normal Plasma manufactured by George King Bio-Medical, Inc. (N = 1), and CRYOcheck Pooled Normal Plasma manufactured by Precision BioLogic Incorporated (N = 1) were used.

- As an LA-positive specimen (LA), Positive Lupus Anticoagulant Plasma manufactured by George King Bio-Medical, Inc. (N = 10) was used.
- As an FVIII inhibitor specimen (VIII#Inh), Factor VIII Deficient with Inhibitor manufactured by George King Bio-Medical, Inc. (N = 6) was used.
- As an FVIII-deficient plasma (HA) and an FIX-deficient plasma (HB), Factor VIII Deficient and Factor IX Deficient manufactured by George King Bio-Medical, Inc. (each N = 1) were used. Assuming that FVIII activities of PNP (citric acid-added pool plasma obtained from normal subjects) and HA are 100% and 0% respectively, PNP and HA were mixed to prepare specimens of an FVIII activity series with FVIII activities of 50%, 25%, 10%, 5%, 2.5%, 1%, 0.75%, 0.5%, 0.25%, and 0.1% (N = 1 at each activity). In a similar manner, assuming that FIX activities of PNP (citric acid-added pool plasma obtained from normal subjects) and HB are 100% and 0% respectively, PNP and HB were mixed to prepare specimens of an FIX activity series with FIX activities of 50%, 25%, 10%, 5%, 2.5%, 1%, 0.75%, 0.5%, 0.25%, and 0.1% (N = 1 at each activity).
- Specimens of a heparin concentration series were prepared by adding heparin (5000 units/5 mL of heparin sodium injection "MOCHIDA" manufactured by MOCHIDA PHARMACEUTICAL CO., LTD.) to PNP (citric acid-added pool plasma obtained from normal subjects) so that heparin concentrations formed a 10-stage concentration series from 0.1 units/mL to 1.0 units/mL at intervals of 0.1 units/mL (N = 1 at each concentration).

1.2) Coagulation reaction measurement

Coagpia APTT-N (manufactured by SEKISUI MEDICAL CO., LTD.) which is a reagent for APTT measurement was used as a reagent for measurement and Coagpia APTT-N Calcium Chloride Solution (manufactured by SEKISUI MEDICAL CO., LTD.) was used as a calcium chloride solution. A coagulation reaction measurement of samples including a specimen was performed using an Automated Coagulation Analyzer CP3000 (manufactured by SEKISUI MEDICAL CO., LTD.). After heating 50 $\mu$L of a specimen in a cuvette at 37°C for 45 seconds, 50 $\mu$L of the reagent for measurement at approximately 37°C was added and, after a lapse of 171 seconds, 50 $\mu$L of a 25 mM calcium chloride solution was added to start a coagulation reaction. The reaction was performed at 37°C. In the measurement of the coagulation reaction, the cuvette was irradiated with light with a wavelength of 660 nm from an LED light source, and a scattered light quantity of light scattered 90 degrees to the side was measured at 0.1-second intervals. The measurement time was set to 360 seconds.

1.3) Acquisition of reaction R(i) and reaction rate V(i)

After performing smoothing including denoising with respect to photometry data from each specimen, zero point adjustment was performed so that a scattered light quantity at a time point of start of photometry equaled zero to create reaction R(i). A first derivative V(i) was calculated from R(i).

1.4) Acquisition of first data and APTT measurement

An APTT of each specimen was measured by a percent method. In other words, a time point where R(i) reached a maximum value Rmax within the measurement time was detected as a coagulation reaction end point E, and a time point where R(i) reached 50% of R(E) was calculated and determined as the APTT.
Figure 8 shows the APTT of each specimen. Data in the drawing are displayed shifted in a direction of an axis of ordinate in accordance with a type of the specimen and a specimen number. Dotted lines in the drawing represent an upper limit (39 seconds) of a range in which the coagulation time is determined to be normal (no prolongation). All of the normal specimens (PNP) had normal coagulation times. Both the LA-positive specimen and the FVIII inhibitor specimen (LA and VIII#Inh in the drawing) had prolonged coagulation times. While the FVIII activity series specimens, the FIX activity series specimens, and the heparin concentration series specimens (FVIII, FIX, and Heparin in the drawing) exhibited trends of a prolonged coagulation time as activity falls or heparin concentration rises, coagulation times of specimens with high activity or low heparin concentration were within a normal range. These results revealed that, specimens with mild coagulation abnormality factors are present among specimens conventionally determined to be normal based on coagulation times. Conventional methods in which specimens having an abnormality in their coagulation times are selectively subjected to a cross-mixing test or other differentiation methods are unable to detect such specimens with mild coagulation abnormality factors.

1.5) Acquisition of second data

A maximum value Vmax of V(i) and a time VmaxT where V(i) = Vmax is satisfied were determined for each specimen. Defining $X_k$ as Vmax $\times$ S% (where S increases in 20 stages at 5% intervals from 3% to 98%), 20 $p_k$ ($p_1$, ...,

$p_{20}$) satisfying V(i) = $X_k$ before VmaxT and 20 $q_k$ ($q_1$, ..., $q_{20}$) satisfying V(i) = $X_k$ after VmaxT were detected (refer to Figure 3). The following parameters were calculated from obtained $p_k$ and $q_k$.

Pre-Ave: average value of $p_k$ ($p_1$, ..., $p_{20}$)
Post-Ave: average value of $q_{kk}$ ($q_1$, ..., $q_{20}$)
All-Ave: average value of $p_k$ ($p_1$, ..., $p_{20}$) and $q_{kk}$ ($q_1$, ..., $q_{20}$)
Pre-SD: standard deviation of $p_k$ ($p_1$, ..., $p_{20}$)
Post-SD: standard deviation of $q_{kk}$ ($q_1$, ..., $q_{20}$)
Pre-CV: coefficient of variation of $p_k$ ($p_1$, ..., $p_{20}$) (%)
Post-CV: coefficient of variation of $q_{kk}$ ($q_1$, ..., q20) (%)
$M_k$: ($p_k$ + $q_k$)/2 (where k is any integer from 1 to 20)
$W_k$: $q_k$ - $p_k$ (where k is any integer from 1 to 20)
Pre-post average difference: (post-Ave - pre-Ave)/all-Ave
Pre-post SD ratio: post-SD/pre-SD
Distortion index: coefficient of variation of $M_k$ ($M_1$, ..., $M_{20}$) (%)
Peakedness index: (sum of $W_1$ to $W_{10}$)/(sum of $W_{11}$ to $W_{20}$)

1.6) Relationship between coagulation abnormality and parameter

1.6.1) Pre-CV and post-CV

Figure 9A is a two-dimensional plot of the pre-CV and the post-CV of each specimen. A trend was observed in which plots exhibited different distributions dependent on a specimen type or, in other words, a type of a coagulation abnormality factor of the specimen. In order to convert a plot position into a relative value, average values of the pre-CV and the post-CV were respectively obtained with respect to the six normal specimens (PNP) and adopted as a reference_pre-CV and a reference_post-CV and, subsequently, a relative_pre-CV and a relative_post-CV of each specimen were obtained using the following equations.

$$\text{Relative\_pre-CV} = (\text{pre-CV of subject specimen}/\text{reference\_pre-CV}) - 1$$

$$\text{Relative\_post-CV} = (\text{post-CV of subject specimen}/\text{reference\_post-CV}) - 1$$

Figure 9B shows a two-dimensional plot of the obtained relative_pre-CV and relative_post-CV. In Figure 9B, a reference point (reference_pre-CV, reference_post-CV) is positioned at an origin (0, 0). Therefore, Figure 9B corresponds to a diagram in which all plots have been shifted so that the reference point overlaps with the origin. Each plot in Figure 9B indicates a relative position of the pre-CV and the post-CV of each specimen with respect to the reference point.

Next, a standard deviation interval (SDI) of the pre-CV and the post-CV of each specimen was obtained using the following equations.

$$\text{SDI} = (\alpha - \beta) \div \gamma,$$

wherein

$\alpha$: value of objective parameter (pre-CV or post-CV) from subject specimen
$\beta$: average value of value of objective parameter (pre-CV or post-CV) from six normal specimens (PNP)
$\gamma$: standard deviation of value of objective parameter (pre-CV or post-CV) from six normal specimens (PNP)
Figure 9C shows a two-dimensional plot of the obtained SDI of the pre-CV and SDI of the post-CV.

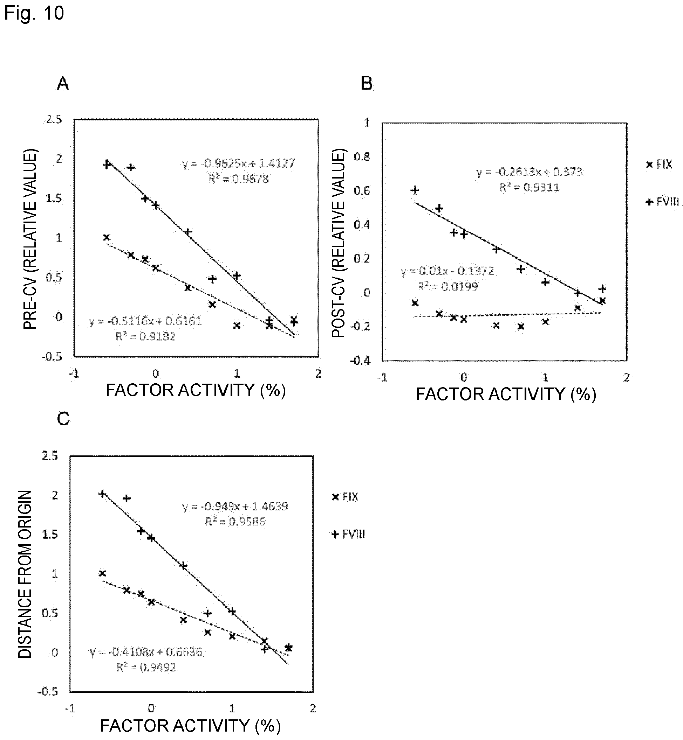

Figure 10 shows a relationship between factor activity (logarithmic value) and the relative_pre-CV and the relative_post-CV in the FVIII activity series specimens and the FIX activity series specimens (FVIII and FIX in the drawings). As shown in Figures 10A and 10B, the FVIII activity had a strong correlation with both the relative_pre-CV and the relative_post-CV, and the FIX activity had a strong correlation with the relative_pre-CV. In addition, as shown in Figure 10C, distances of points (relative_pre-CV, relative_post-CV) on the two-dimensional plot shown in Figure 9B from the origin had a strong correlation with both the FVIII activity and the FIX activity.

Table 1-1 shows the APTT, the pre-CV, the post-CV, the relative_pre-CV, and the relative_post-CV of each specimen type. In Table 1-1, the relative_pre-CV is denoted as *pre-CV and the relative_post-CV is denoted as *post-CV. The APTT in Table 1-1 is represented by bold black letters against a pale gray background when exceeding an upper limit (39 seconds) of a normal range. In addition, the pre-CV, the post-CV, the relative_pre-CV, and the relative_post-CV in Table 1-1 are represented by white letters against a dark gray background when a value of a specimen is lower than a minimum value of PNP, represented by bold black letters against a pale gray background when a value of a specimen is higher than a maximum value of PNP, and represented by black letters against a white background when a value of a specimen is within a value range from PNP.

Table 1-2 shows the APTT, the pre-CV, the post-CV, the SDI of the pre-CV, and the SDI of the post-CV of each specimen type. In Table 1-2, the SDI of the pre-CV is denoted as *pre-CV and the SDI of the post-CV is denoted as *post-CV. Character styles of the APTT, the pre-CV, and the post-CV in Table 1-2 have a same meaning as in Table 1-1. In addition, the *pre-CV and the *post-CV in Table 1-2 are represented by white letters against a dark gray background when the SDI is lower than -3, represented by bold black letters against a pale gray background when the SDI is higher than 3, and represented by black letters against a white background when the SDI is within ±3.

[Table 1-1]

| Specimen type | APTT | Pre-CV | Post-CV | *Pre-CV | *Post-CV |
|---|---|---|---|---|---|
| PNP | 27.9 | 5.1 | 13.0 | 0.08 | 0.05 |
| PNP | 29.9 | 4.3 | 12.5 | -0.08 | 0.01 |
| PNP | 29.1 | 4.3 | 11.2 | -0.08 | -0.09 |
| PNP | 27.6 | 5.2 | 12.3 | 0.10 | 0.00 |
| PNP | 27.5 | 4.6 | 12.7 | -0.02 | 0.03 |
| PNP | 26.1 | 4.7 | 12.3 | 0.00 | 0.00 |

| Specimen type | APTT | Pre-CV | Post-CV | *Pre-CV | *Post-CV |
|---|---|---|---|---|---|
| LA | 54.5 | 3.2 | 29.5 | -0.32 | 1.39 |
| LA | 54.1 | 7.5 | 17.1 | 0.60 | 0.39 |
| LA | 53.6 | 8.8 | 16.8 | 0.86 | 0.36 |
| LA | 64.7 | 10.9 | 16.0 | 1.31 | 0.30 |
| LA | 51.5 | 3.3 | 28.2 | -0.30 | 1.28 |
| LA | 68.0 | 10.5 | 15.1 | 1.22 | 0.22 |
| LA | 97.7 | 2.8 | 19.3 | -0.40 | 0.57 |
| LA | 47.2 | 4.3 | 14.8 | -0.10 | 0.20 |
| LA | 54.8 | 3.2 | 28.0 | -0.32 | 1.27 |
| LA | 100.2 | 2.5 | 18.2 | -0.48 | 0.47 |

| Specimen type | APTT | Pre-CV | Post-CV | *Pre-CV | *Post-CV |
|---|---|---|---|---|---|
| VIII_Inh | 144.0 | 6.3 | 30.3 | 0.33 | 1.45 |
| VIII_inh | 139.7 | 5.5 | 33.4 | 0.17 | 1.70 |
| VIII_inh | 129.1 | 5.2 | 33.0 | 0.10 | 1.67 |
| VIII_Inh | 156.0 | 5.3 | 25.8 | 0.12 | 1.09 |
| VIII_Inh | 133.6 | 4.6 | 39.0 | -0.02 | 2.16 |
| VIII_Inh | 132.5 | 5.0 | 36.6 | 0.06 | 1.96 |

| Specimen type | Activity | APTT | Pre-CV | Post-CV | *Pre-CV | *Post-CV |
|---|---|---|---|---|---|---|
| FVIII | 50 | 32.4 | 4.4 | 12.6 | -0.07 | 0.02 |
| FVIII | 25 | 37.7 | 4.5 | 12.3 | -0.04 | 0.00 |
| FVIII | 10 | 45.5 | 7.2 | 13.1 | 0.52 | 0.06 |
| FVIII | 5 | 51.9 | 7.0 | 14.0 | 0.48 | 0.14 |
| FVIII | 2.5 | 59.7 | 9.8 | 15.5 | 1.08 | 0.25 |
| FVIII | 1 | 70.3 | 11.4 | 16.6 | 1.41 | 0.34 |
| FVIII | 0.75 | 73.1 | 11.8 | 16.7 | 1.50 | 0.35 |
| FVIII | 0.5 | 81.3 | 13.7 | 18.5 | 1.90 | 0.50 |
| FVIII | 0.25 | 89.7 | 13.8 | 19.8 | 1.93 | 0.60 |
| FVIII | 0.1 | 120.0 | 5.0 | 35.2 | 0.07 | 1.85 |

| Specimen type | Activity | APTT | Pre-CV | Post-CV | *Pre-CV | *Post-CV |
|---|---|---|---|---|---|---|
| FIX | 50 | 29.0 | 4.6 | 11.8 | -0.03 | -0.05 |
| FIX | 25 | 31.8 | 4.2 | 11.2 | -0.11 | -0.09 |
| FIX | 10 | 36.5 | 4.2 | 10.2 | -0.11 | -0.17 |
| FIX | 5 | 40.9 | 5.5 | 9.8 | 0.16 | -0.20 |
| FIX | 2.5 | 45.6 | 6.5 | 10.0 | 0.37 | -0.19 |
| FIX | 1 | 53.4 | 7.6 | 10.4 | 0.62 | -0.16 |
| FIX | 0.75 | 54.5 | 8.2 | 10.5 | 0.73 | -0.15 |
| FIX | 0.5 | 58.2 | 8.4 | 10.8 | 0.78 | -0.13 |
| FIX | 0.25 | 64.0 | 9.5 | 11.6 | 1.01 | -0.06 |
| FIX | 0.1 | 81.3 | 12.3 | 14.6 | 1.60 | 0.18 |

| Specimen type | Unit | APTT | Pre-CV | Post-CV | *Pre-CV | *Post-CV |
|---|---|---|---|---|---|---|
| Heparin | 0.1 | 30.5 | 4.4 | 11.2 | -0.06 | -0.09 |
| Heparin | 0.2 | 36.1 | 4.1 | 10.1 | -0.13 | -0.18 |
| Heparin | 0.3 | 43.5 | 4.1 | 9.1 | -0.12 | -0.26 |
| Heparin | 0.4 | 54.3 | 4.3 | 8.1 | -0.08 | -0.34 |
| Heparin | 0.5 | 64.7 | 4.6 | 7.7 | -0.02 | -0.38 |
| Heparin | 0.6 | 76.9 | 4.8 | 7.1 | 0.02 | -0.42 |
| Heparin | 0.7 | 97.0 | 5.1 | 6.6 | 0.09 | -0.47 |
| Heparin | 0.8 | 104.3 | 5.5 | 6.6 | 0.17 | -0.46 |
| Heparin | 0.9 | 126.3 | 5.5 | 6.3 | 0.17 | -0.49 |
| Heparin | 1.0 | 146.2 | 6.0 | 6.1 | 0.27 | -0.50 |

[Table 1-2]

| Specimen type | APTT | Pre-CV | Post-CV | *Pre-CV | *Post-CV |
|---|---|---|---|---|---|
| PNP | 27.9 | 5.1 | 13.0 | 1.0 | 1.1 |
| PNP | 29.9 | 4.3 | 12.5 | -1.1 | 0.3 |
| PNP | 29.1 | 4.3 | 11.2 | -1.0 | -1.8 |
| PNP | 27.6 | 5.2 | 12.3 | 1.3 | 0.0 |
| PNP | 27.5 | 4.6 | 12.7 | -0.2 | 0.6 |
| PNP | 26.1 | 4.7 | 12.3 | 0.1 | -0.1 |

| Specimen type | APTT | Pre-CV | Post-CV | *Pre-CV | *Post-CV |
|---|---|---|---|---|---|
| LA | 54.5 | 3.2 | 29.5 | -4.2 | 28.9 |
| LA | 54.1 | 7.5 | 17.1 | 7.8 | 8.0 |
| LA | 53.6 | 8.8 | 16.8 | 11.3 | 7.5 |
| LA | 64.7 | 10.9 | 16.0 | 17.1 | 6.2 |
| LA | 51.5 | 3.3 | 28.2 | -3.9 | 26.6 |
| LA | 68.0 | 10.5 | 15.1 | 15.9 | 4.6 |
| LA | 97.7 | 2.8 | 19.3 | -5.2 | 11.8 |
| LA | 47.2 | 4.3 | 14.8 | -1.3 | 4.1 |
| LA | 54.8 | 3.2 | 28.0 | -4.2 | 26.3 |
| LA | 100.2 | 2.5 | 18.2 | -6.2 | 9.8 |

| Specimen type | APTT | Pre-CV | Post-CV | *Pre-CV | *Post-CV |
|---|---|---|---|---|---|
| VIII_Inh | 144.0 | 6.3 | 30.3 | 4.3 | 30.1 |
| VIII_inh | 139.7 | 5.5 | 33.4 | 2.2 | 35.3 |
| VIII_inh | 129.1 | 5.2 | 33.0 | 1.3 | 34.6 |
| VIII_Inh | 156.0 | 5.3 | 25.8 | 1.6 | 22.6 |
| VIII_Inh | 133.6 | 4.6 | 39.0 | -0.2 | 44.7 |
| VIII_Inh | 132.5 | 5.0 | 36.6 | 0.8 | 40.7 |

| Specimen type | Activity | APTT | Pre-CV | Post-CV | *Pre-CV | *Post-CV |
|---|---|---|---|---|---|---|
| FVIII | 50 | 32.4 | 4.4 | 12.6 | -0.9 | 0.5 |
| FVIII | 25 | 37.7 | 4.5 | 12.3 | -0.6 | 0.0 |
| FVIII | 10 | 45.5 | 7.2 | 13.1 | 6.8 | 1.3 |
| FVIII | 5 | 51.9 | 7.0 | 14.0 | 6.2 | 2.8 |
| FVIII | 2.5 | 59.7 | 9.8 | 15.5 | 14.1 | 5.2 |
| FVIII | 1 | 70.3 | 11.4 | 16.6 | 18.4 | 7.1 |
| FVIII | 0.75 | 73.1 | 11.8 | 16.7 | 19.6 | 7.3 |
| FVIII | 0.5 | 81.3 | 13.7 | 18.5 | 24.8 | 10.3 |
| FVIII | 0.25 | 89.7 | 13.8 | 19.8 | 25.2 | 12.5 |
| FVIII | 0.1 | 120.0 | 5.0 | 35.2 | 0.9 | 38.3 |

| Specimen type | Activity | APTT | Pre-CV | Post-CV | *Pre-CV | *Post-CV |
|---|---|---|---|---|---|---|
| FIX | 50 | 29.0 | 4.6 | 11.8 | -0.4 | -1.0 |
| FIX | 25 | 31.8 | 4.2 | 11.2 | -1.5 | -1.8 |
| FIX | 10 | 36.5 | 4.2 | 10.2 | -1.4 | -3.6 |
| FIX | 5 | 40.9 | 5.5 | 9.8 | 2.1 | -4.2 |
| FIX | 2.5 | 45.6 | 6.5 | 10.0 | 4.8 | -4.0 |
| FIX | 1 | 53.4 | 7.6 | 10.4 | 8.1 | -3.2 |
| FIX | 0.75 | 54.5 | 8.2 | 10.5 | 9.5 | -3.1 |
| FIX | 0.5 | 58.2 | 8.4 | 10.8 | 10.2 | -2.6 |
| FIX | 0.25 | 64.0 | 9.5 | 11.6 | 13.1 | -1.2 |
| FIX | 0.1 | 81.3 | 12.3 | 14.6 | 20.9 | 3.8 |

| Specimen type | Unit | APTT | Pre-CV | Post-CV | *Pre-CV | *Post-CV |
|---|---|---|---|---|---|---|
| Heparin | 0.1 | 30.5 | 4.4 | 11.2 | -0.8 | -1.9 |
| Heparin | 0.2 | 36.1 | 4.1 | 10.1 | -1.6 | -3.7 |
| Heparin | 0.3 | 43.5 | 4.1 | 9.1 | -1.6 | -5.4 |
| Heparin | 0.4 | 54.3 | 4.3 | 8.1 | -1.1 | -7.1 |
| Heparin | 0.5 | 64.7 | 4.6 | 7.7 | -0.3 | -7.8 |
| Heparin | 0.6 | 76.9 | 4.8 | 7.1 | 0.2 | -8.7 |
| Heparin | 0.7 | 97.0 | 5.1 | 6.6 | 1.1 | -9.7 |
| Heparin | 0.8 | 104.3 | 5.5 | 6.6 | 2.2 | -9.6 |
| Heparin | 0.9 | 126.3 | 5.5 | 6.3 | 2.3 | -10.1 |
| Heparin | 1.0 | 146.2 | 6.0 | 6.1 | 3.6 | -10.4 |

1.6.2) Distortion index and peakedness index

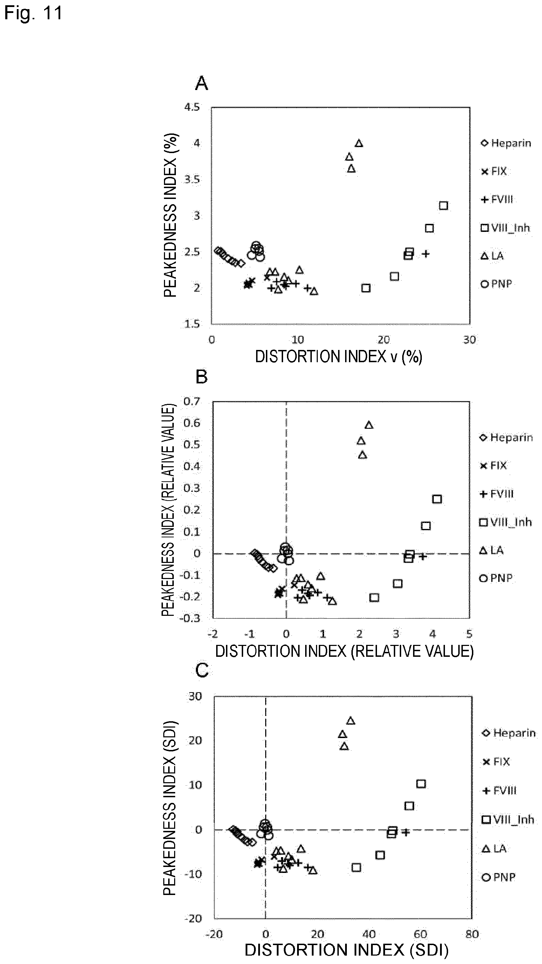

Figure 11A is a two-dimensional plot of a distortion index and a peakedness index of each specimen. A trend was observed in which plots exhibited different distributions dependent on a specimen type or, in other words, a type of a coagulation abnormality factor of the specimen. In order to convert a plot position into a relative value, average values of the distortion index and the peakedness index were respectively obtained with respect to the six normal specimens (PNP) and adopted as a reference_distortion index and a reference_peakedness index and, subsequently, a relative_distortion index and a relative_peakedness index of each specimen were obtained using the following equations.

```
Relative_distortion index = (distortion index of

subject specimen/reference_distortion index) - 1
```

```
Relative_peakedness index = (peakedness index of

subject specimen/reference_peakedness index) - 1
```

Figure 11B shows a two-dimensional plot of the obtained relative_distortion index and relative_peakedness index. In Figure 11B, a reference point (reference_distortion index, reference_peakedness index) is positioned at an origin (0, 0) . Therefore, Figure 11B corresponds to a diagram in which all plots have been shifted so that the reference point overlaps with the origin. Each plot in Figure 11B indicates a relative position of the distortion index and the peakedness index of each specimen with respect to the reference point.

Next, the SDI of the distortion index and the peakedness index of each specimen was obtained by the same procedure as in 1.6.1). Figure 11C shows a two-dimensional plot of the obtained SDI of the distortion index and the SDI of the peakedness index.

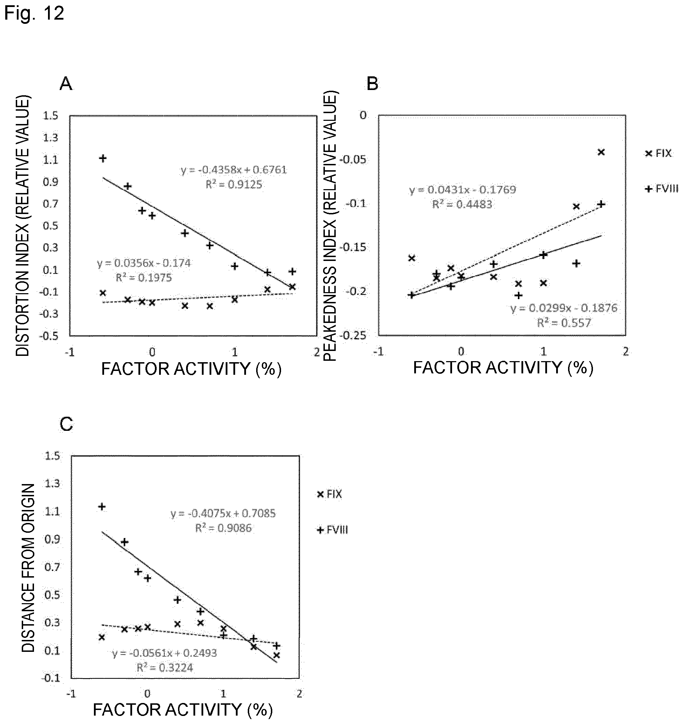

Figure 12 shows a relationship between factor activity (logarithmic value) and the relative_distortion index and the relative_peakedness index in the FVIII activity series specimens and the FIX activity series specimens (FVIII and FIX in the drawings). As shown in Figures 12A and 12B, the FVIII activity had a strong correlation with the relative_distortion index. In addition, as shown in Figure 12C, distances of points (relative_distortion index, relative_peakedness index) on the two-dimensional plot shown in Figure 11B from the origin had a strong correlation with the FVIII activity.

Table 2-1 shows the APTT, the distortion index, the peakedness index, the relative_distortion index, and the relative_peakedness index of each specimen type. In Table 2-1, the relative_distortion index is denoted as *distortion index and the relative_peakedness index is denoted as *peakedness index. Table 2-2 shows the APTT, the distortion index, the peakedness index, the SDI of the distortion index, and the SDI of the peakedness index of each specimen type. In Table 2-2, the SDI of the distortion index is denoted as *distortion index and the SDI of the peakedness index is denoted as *peakedness index. Character styles in Table 2-1 and Table 2-2 respectively have a same meaning as in Tables 1-1 and 1-2.

[Table 2-1]

| Specimen type | APTT | Distortion index | Peakedness index | *Distortion index | *Peakedness index |
|---|---|---|---|---|---|
| PNP | 27.9 | 5.7 | 2.4 | 0.08 | -0.03 |
| PNP | 29.9 | 5.5 | 2.5 | 0.04 | 0.02 |
| PNP | 29.1 | 4.7 | 2.5 | -0.11 | -0.02 |
| PNP | 27.6 | 5.0 | 2.5 | -0.04 | 0.01 |
| PNP | 27.5 | 5.5 | 2.5 | 0.05 | 0.00 |
| PNP | 26.1 | 5.2 | 2.6 | -0.02 | 0.03 |

| Specimen type | APTT | Distortion index | Peakedness index | *Distortion index | *Peakedness index |
|---|---|---|---|---|---|
| LA | 54.5 | 17.1 | 4.0 | 2.25 | 0.59 |
| LA | 54.1 | 8.9 | 2.1 | 0.69 | -0.16 |
| LA | 53.6 | 8.4 | 2.2 | 0.60 | -0.14 |
| LA | 64.7 | 7.4 | 2.2 | 0.40 | -0.11 |
| LA | 51.5 | 16.2 | 3.7 | 2.08 | 0.45 |
| LA | 68.0 | 6.8 | 2.2 | 0.29 | -0.11 |
| LA | 97.7 | 11.9 | 2.0 | 1.26 | -0.22 |
| LA | 47.2 | 7.7 | 2.0 | 0.47 | -0.21 |
| LA | 54.8 | 16.0 | 3.8 | 2.04 | 0.52 |
| LA | 100.2 | 10.2 | 2.3 | 0.94 | -0.10 |

| Specimen type | APTT | Distortion index | Peakedness index | *Distortion index | *Peakedness index |
|---|---|---|---|---|---|
| VIII_Inh | 144.0 | 21.3 | 2.2 | 3.04 | -0.14 |
| VIII_inh | 139.7 | 23.0 | 2.5 | 3.37 | 0.00 |
| VIII_inh | 129.1 | 22.8 | 2.5 | 3.34 | -0.02 |
| VIII_Inh | 156.0 | 17.9 | 2.0 | 2.41 | -0.20 |
| VIII_Inh | 133.6 | 26.9 | 3.1 | 4.11 | 0.25 |
| VIII_Inh | 132.5 | 25.3 | 2.8 | 3.81 | 0.13 |

| Specimen type | Activity | APTT | Distortion index | Peakedness index | *Distortion index | *Peakedness index |
|---|---|---|---|---|---|---|
| FVIII | 50.0 | 32.4 | 5.7 | 2.3 | 0.09 | -0.10 |
| FVIII | 25.0 | 37.7 | 5.7 | 2.1 | 0.08 | -0.17 |
| FVIII | 10.0 | 45.5 | 6.0 | 2.1 | 0.14 | -0.16 |
| FVIII | 5.0 | 51.9 | 7.0 | 2.0 | 0.32 | -0.20 |
| FVIII | 2.5 | 59.7 | 7.5 | 2.1 | 0.43 | -0.17 |
| FVIII | 1.0 | 70.3 | 8.4 | 2.0 | 0.59 | -0.18 |
| FVIII | 0.75 | 73.1 | 8.6 | 2.0 | 0.64 | -0.19 |
| FVIII | 0.5 | 81.3 | 9.8 | 2.1 | 0.86 | -0.18 |
| FVIII | 0.25 | 89.7 | 11.1 | 2.0 | 1.11 | -0.20 |
| FVIII | 0.1 | 120.0 | 24.9 | 2.5 | 3.73 | -0.01 |

| Specimen type | Activity | APTT | Distortion index | Peakedness index | *Distortion index | *Peakedness index |
|---|---|---|---|---|---|---|
| FIX | 50.0 | 29.0 | 5.0 | 2.4 | -0.05 | -0.04 |
| FIX | 25.0 | 31.8 | 4.9 | 2.3 | -0.08 | -0.10 |
| FIX | 10.0 | 36.5 | 4.4 | 2.0 | -0.17 | -0.19 |
| FIX | 5.0 | 40.9 | 4.1 | 2.0 | -0.23 | -0.19 |
| FIX | 2.5 | 45.6 | 4.1 | 2.1 | -0.22 | -0.18 |
| FIX | 1.0 | 53.4 | 4.2 | 2.1 | -0.20 | -0.18 |
| FIX | 0.75 | 54.5 | 4.3 | 2.1 | -0.19 | -0.17 |
| FIX | 0.5 | 58.2 | 4.4 | 2.0 | -0.17 | -0.19 |
| FIX | 0.25 | 64.0 | 4.7 | 2.1 | -0.11 | -0.16 |
| FIX | 0.1 | 81.3 | 6.4 | 2.1 | 0.22 | -0.15 |

| Specimen type | Unit | APTT | Distortion index | Peakedness index | *Distortion index | *Peakedness index |
|---|---|---|---|---|---|---|
| Heparin | 0.1 | 30.5 | 4.6 | 2.5 | -0.12 | 0.00 |
| Heparin | 0.2 | 36.1 | 4.1 | 2.4 | -0.23 | -0.05 |
| Heparin | 0.3 | 43.5 | 3.4 | 2.3 | -0.35 | -0.07 |
| Heparin | 0.4 | 54.3 | 2.7 | 2.4 | -0.48 | -0.06 |
| Heparin | 0.5 | 64.7 | 2.3 | 2.4 | -0.56 | -0.06 |
| Heparin | 0.6 | 76.9 | 1.9 | 2.4 | -0.64 | -0.04 |
| Heparin | 0.7 | 97.0 | 1.4 | 2.5 | -0.74 | -0.02 |
| Heparin | 0.8 | 104.3 | 1.2 | 2.5 | -0.77 | -0.01 |
| Heparin | 0.9 | 126.3 | 1.1 | 2.5 | -0.80 | 0.00 |
| Heparin | 1.0 | 146.2 | 0.7 | 2.5 | -0.86 | 0.00 |

[Table 2-2]

| Specimen type | APTT | Distortion index | Peakedness index | *Distortion index | *Peakedness index |
|---|---|---|---|---|---|
| PNP | 27.9 | 5.7 | 2.4 | 1.1 | -1.4 |
| PNP | 29.9 | 5.5 | 2.5 | 0.6 | 0.6 |
| PNP | 29.1 | 4.7 | 2.5 | -1.6 | -0.9 |
| PNP | 27.6 | 5.0 | 2.5 | -0.6 | 0.5 |
| PNP | 27.5 | 5.5 | 2.5 | 0.7 | -0.1 |
| PNP | 26.1 | 5.2 | 2.6 | -0.2 | 1.2 |

| Specimen type | APTT | Distortion index | Peakedness index | *Distortion index | *Peakedness index |
|---|---|---|---|---|---|
| LA | 54.5 | 17.1 | 4.0 | 32.9 | 24.6 |
| LA | 54.1 | 8.9 | 2.1 | 10.1 | -6.6 |
| LA | 53.6 | 8.4 | 2.2 | 8.8 | -5.9 |
| LA | 64.7 | 7.4 | 2.2 | 5.8 | -4.7 |
| LA | 51.5 | 16.2 | 3.7 | 30.4 | 18.8 |
| LA | 68.0 | 6.8 | 2.2 | 4.2 | -4.7 |
| LA | 97.7 | 11.9 | 2.0 | 18.4 | -9.1 |
| LA | 47.2 | 7.7 | 2.0 | 6.8 | -8.7 |
| LA | 54.8 | 16.0 | 3.8 | 29.8 | 21.5 |
| LA | 100.2 | 10.2 | 2.3 | 13.7 | -4.2 |

| Specimen type | APTT | Distortion index | Peakedness index | *Distortion index | *Peakedness index |
|---|---|---|---|---|---|
| VIII_Inh | 144.0 | 21.3 | 2.2 | 44.3 | -5.7 |
| VIII_inh | 139.7 | 23.0 | 2.5 | 49.2 | -0.1 |
| VIII_inh | 129.1 | 22.8 | 2.5 | 48.7 | -0.9 |
| VIII_Inh | 156.0 | 17.9 | 2.0 | 35.1 | -8.4 |
| VIII_Inh | 133.6 | 26.9 | 3.1 | 60.1 | 10.4 |
| VIII_Inh | 132.5 | 25.3 | 2.8 | 55.6 | 5.3 |

| Specimen type | Activity | APTT | Distortion index | Peakedness index | *Distortion index | *Peakedness index |
|---|---|---|---|---|---|---|
| FVIII | 50 | 32.4 | 5.7 | 2.3 | 1.3 | -4.2 |
| FVIII | 25 | 37.7 | 5.7 | 2.1 | 1.2 | -7.0 |
| FVIII | 10 | 45.5 | 6.0 | 2.1 | 2.0 | -6.6 |
| FVIII | 5 | 51.9 | 7.0 | 2.0 | 4.7 | -8.5 |
| FVIII | 2.5 | 59.7 | 7.5 | 2.1 | 6.3 | -7.0 |
| FVIII | 1 | 70.3 | 8.4 | 2.0 | 8.7 | -7.6 |
| FVIII | 0.75 | 73.1 | 8.6 | 2.0 | 9.3 | -8.0 |
| FVIII | 0.5 | 81.3 | 9.8 | 2.1 | 12.6 | -7.4 |
| FVIII | 0.25 | 89.7 | 11.1 | 2.0 | 16.3 | -8.5 |
| FVIII | 0.1 | 120.0 | 24.9 | 2.5 | 54.4 | -0.6 |

| Specimen type | Activity | APTT | Distortion index | Peakedness index | *Distortion index | *Peakedness index |
|---|---|---|---|---|---|---|
| FIX | 50 | 29.0 | 5.0 | 2.4 | -0.8 | -1.7 |
| FIX | 25 | 31.8 | 4.9 | 2.3 | -1.1 | -4.3 |
| FIX | 10 | 36.5 | 4.4 | 2.0 | -2.5 | -7.9 |
| FIX | 5 | 40.9 | 4.1 | 2.0 | -3.3 | -7.9 |
| FIX | 2.5 | 45.6 | 4.1 | 2.1 | -3.3 | -7.6 |
| FIX | 1 | 53.4 | 4.2 | 2.1 | -2.9 | -7.5 |
| FIX | 0.75 | 54.5 | 4.3 | 2.1 | -2.8 | -7.2 |
| FIX | 0.5 | 58.2 | 4.4 | 2.0 | -2.5 | -7.7 |
| FIX | 0.25 | 64.0 | 4.7 | 2.1 | -1.6 | -6.7 |
| FIX | 0.1 | 81.3 | 6.4 | 2.1 | 3.3 | -6.0 |

| Specimen type | Unit | APTT | Distortion index | Peakedness index | *Distortion index | *Peakedness index |
|---|---|---|---|---|---|---|
| Heparin | 0.1 | 30.5 | 4.6 | 2.5 | -1.8 | 0.0 |
| Heparin | 0.2 | 36.1 | 4.1 | 2.4 | -3.4 | -2.0 |
| Heparin | 0.3 | 43.5 | 3.4 | 2.3 | -5.1 | -2.8 |
| Heparin | 0.4 | 54.3 | 2.7 | 2.4 | -7.0 | -2.7 |
| Heparin | 0.5 | 64.7 | 2.3 | 2.4 | -8.1 | -2.3 |
| Heparin | 0.6 | 76.9 | 1.9 | 2.4 | -9.4 | -1.7 |
| Heparin | 0.7 | 97.0 | 1.4 | 2.5 | -10.8 | -1.0 |
| Heparin | 0.8 | 104.3 | 1.2 | 2.5 | -11.2 | -0.4 |
| Heparin | 0.9 | 126.3 | 1.1 | 2.5 | -11.7 | -0.1 |
| Heparin | 1.0 | 146.2 | 0.7 | 2.5 | -12.6 | 0.1 |

23

Figure 13A shows a two-dimensional plot of the relative_distortion index and the relative_peakedness index of an abnormal specimen which has a coagulation abnormality factor but of which an APTT is within a normal range (39 seconds or less) together with plots of PNP. Figure 13B shows a two-dimensional plot of the SDI of the distortion index and the SDI of the peakedness index of the same abnormal specimen together with plots of PNP. Figures 13C and 13D are tables showing the APTT, the distortion index, the peakedness index, *distortion index (relative_distortion index or distortion index SDI), and *peakedness index (relative_peakedness index or peakedness index SDI) of the abnormal specimen shown in Figures 13A and 13B. Character styles in tables of Figures 13C and 13D respectively have a same meaning as in Tables 2-1 and 2-2. The abnormal specimens shown in Figure 13 differed from PNP in at least one of the relative_distortion index and the relative_peakedness index or exhibited a different SDI from PNP.

These results revealed that the relative_distortion index and the relative_peakedness index or the SDIs of the distortion index and the peakedness index can be used as indices for distinguishing abnormal specimens from normal specimens and that the indices are effective in detecting a specimen which actually has an abnormality factor despite its coagulation time being within a normal range. However, with respect to two specimens (specimen of 0.1 units of Heparin and specimen of FIX with 50% activity), the specimens could not be distinguished from PNP using the indices due to proximity of their plots to PNP.

1.6.3) Pre-post average difference and pre-post SD ratio

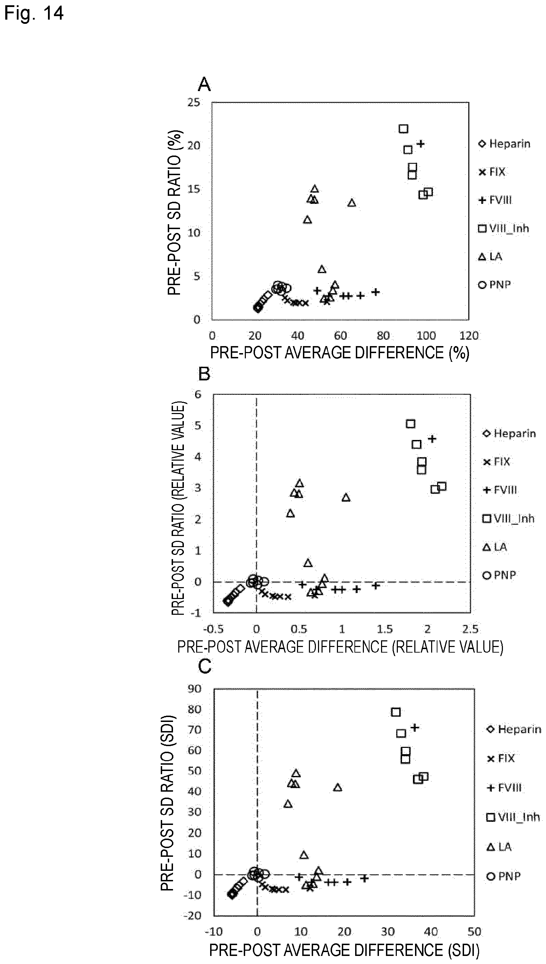

Figure 14A is a two-dimensional plot of a pre-post average difference and a pre-post SD ratio of each specimen. A trend was observed in which plots exhibited different distributions dependent on a specimen type or, in other words, a type of a coagulation abnormality factor of the specimen. In order to convert a plot position into a relative value, average values of the pre-post average difference and the pre-post SD ratio were respectively obtained with respect to the six normal specimens (PNP) and adopted as a reference_pre-post average difference and a reference_pre-post SD ratio and, subsequently, a relative_pre-post average difference and a relative_pre-post SD ratio of each specimen were obtained using the following equations.

```
Relative_pre-post average difference = (pre-post

average difference of subject specimen/reference_pre-post

average difference) - 1


Relative_pre-post SD ratio = (pre-post SD ratio of

subject specimen/reference_pre-post SD ratio) - 1
```

Figure 14B shows a two-dimensional plot of the obtained relative_pre-post average difference and relative_pre-post SD ratio. In Figure 14B, a reference point (reference_pre-post average difference, reference_pre-post SD ratio) is positioned at an origin (0, 0). Therefore, Figure 14B corresponds to a diagram in which all plots have been shifted so that the reference point overlaps with the origin. Each plot in Figure 14B indicates a relative position of the pre-post average difference and the pre-post SD ratio of each specimen with respect to the reference point.

Next, the SDI of the pre-post average difference and the pre-post SD ratio of each specimen was obtained by the same procedure as in 1.6.1). Figure 14C shows a two-dimensional plot of the obtained SDI of the pre-post average difference and SDI of the pre-post SD ratio.

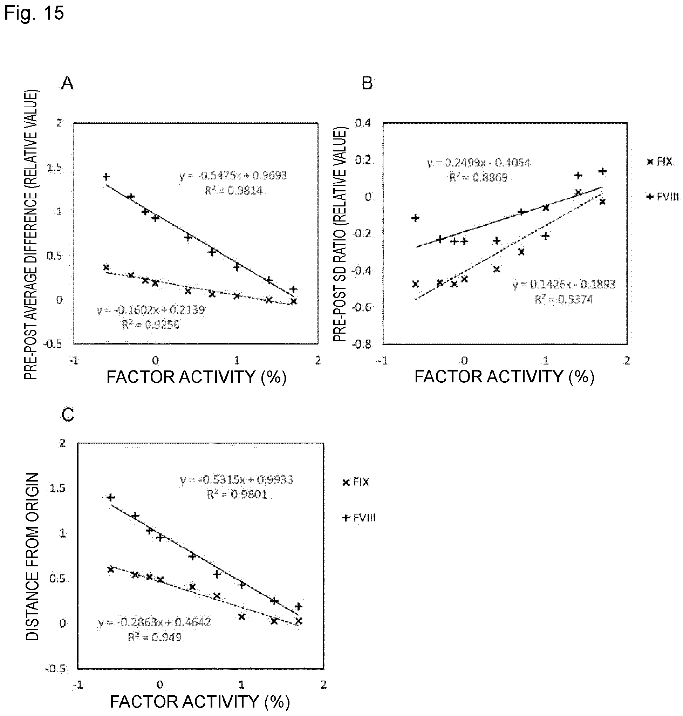

Figure 15 shows a relationship between factor activity (logarithmic value) and the relative_pre-post average difference and the relative_pre-post SD ratio in the FVIII activity series specimens and the FIX activity series specimens (FVIII and FIX in the drawings). As shown in Figures 15A and 15B, the relative_pre-post average difference had a strong correlation with both the FVIII activity and the FIX activity. The relative_pre-post SD ratio had a strong correlation with FVIII activity. In addition, as shown in Figure 15C, distances of points (relative_pre-post average difference, relative_pre-post SD ratio) on the two-dimensional plot shown in Figure 14B from the origin had a strong correlation with both the FVIII activity and the FIX activity.

Table 3-1 shows the APTT, the pre-post average difference (lnA), the pre-post SD ratio (lnB), the relative_pre-post average difference (*lnA), and the relative_pre-post SD ratio (*lnB) of each specimen type. Table 3-2 shows the APTT, the pre-post average difference (lnA), the pre-post SD ratio (lnB), the pre-post average difference SDI (*lnA), and the pre-post SD ratio SDI (*lnB) of each specimen type. Character styles in Table 3-1 and Table 3-2 respectively have a same meaning as in Tables 1-1 and 1-2.

[Table 3-1]

| Specimen type | APTT | InA | InB | *InA | *InB |
|---|---|---|---|---|---|
| PNP | 27.9 | 34.9 | 3.6 | 0.10 | 0.01 |
| PNP | 29.9 | 30.8 | 4.0 | -0.04 | 0.09 |
| PNP | 29.1 | 29.9 | 3.5 | -0.06 | -0.03 |
| PNP | 27.6 | 32.4 | 3.3 | 0.02 | -0.09 |
| PNP | 27.5 | 32.6 | 3.8 | 0.02 | 0.05 |
| PNP | 26.1 | 30.8 | 3.5 | -0.03 | -0.02 |

| Specimen type | APTT | InA | InB | *InA | *InB |
|---|---|---|---|---|---|
| LA | 54.5 | 48.0 | 15.1 | 0.50 | 3.16 |
| LA | 54.1 | 57.3 | 4.1 | 0.80 | 0.13 |
| LA | 53.6 | 56.5 | 3.4 | 0.77 | -0.06 |
| LA | 64.7 | 55.1 | 2.6 | 0.73 | -0.29 |
| LA | 51.5 | 47.7 | 13.8 | 0.49 | 2.82 |
| LA | 68.0 | 52.2 | 2.5 | 0.64 | -0.32 |
| LA | 97.7 | 65.3 | 13.5 | 1.05 | 2.72 |
| LA | 47.2 | 51.2 | 5.9 | 0.60 | 0.62 |
| LA | 54.8 | 46.1 | 14.0 | 0.44 | 2.86 |
| LA | 100.2 | 44.6 | 11.6 | 0.40 | 2.20 |

| Specimen type | APTT | InA | InB | *InA | *InB |
|---|---|---|---|---|---|
| VIII_Inh | 144.0 | 100.9 | 14.7 | 2.16 | 3.06 |
| VIII_inh | 139.7 | 93.4 | 16.6 | 1.93 | 3.59 |
| VIII_inh | 129.1 | 93.5 | 17.6 | 1.93 | 3.85 |
| VIII_Inh | 156.0 | 98.6 | 14.4 | 2.09 | 2.96 |
| VIII_Inh | 133.6 | 89.4 | 22.0 | 1.80 | 5.07 |
| VIII_Inh | 132.5 | 91.5 | 19.6 | 1.87 | 4.40 |

| Specimen type | Activity | APTT | InA | InB | *InA | *InB |
|---|---|---|---|---|---|---|
| FVIII | 50.0 | 32.4 | 35.9 | 4.1 | 0.12 | 0.14 |
| FVIII | 25.0 | 37.7 | 38.9 | 4.0 | 0.22 | 0.12 |
| FVIII | 10.0 | 45.5 | 43.7 | 2.8 | 0.37 | -0.21 |
| FVIII | 5.0 | 51.9 | 49.2 | 3.3 | 0.54 | -0.08 |
| FVIII | 2.5 | 59.7 | 54.4 | 2.8 | 0.70 | -0.24 |
| FVIII | 1.0 | 70.3 | 61.3 | 2.7 | 0.92 | -0.24 |
| FVIII | 0.75 | 73.1 | 63.8 | 2.7 | 1.00 | -0.24 |
| FVIII | 0.5 | 81.3 | 69.3 | 2.8 | 1.17 | -0.23 |
| FVIII | 0.25 | 89.7 | 76.4 | 3.2 | 1.39 | -0.12 |
| FVIII | 0.1 | 120.0 | 97.4 | 20.2 | 2.05 | 4.58 |

| Specimen type | Activity | APTT | InA | InB | *InA | *InB |
|---|---|---|---|---|---|---|
| FIX | 50.0 | 29.0 | 31.5 | 3.5 | -0.01 | -0.02 |
| FIX | 25.0 | 31.8 | 31.9 | 3.7 | 0.00 | 0.02 |
| FIX | 10.0 | 36.5 | 33.3 | 3.4 | 0.04 | -0.06 |
| FIX | 5.0 | 40.9 | 34.0 | 2.5 | 0.07 | -0.30 |
| FIX | 2.5 | 45.6 | 35.2 | 2.2 | 0.10 | -0.39 |
| FIX | 1.0 | 53.4 | 38.0 | 2.0 | 0.19 | -0.45 |
| FIX | 0.75 | 54.5 | 38.9 | 1.9 | 0.22 | -0.47 |
| FIX | 0.5 | 58.2 | 40.8 | 1.9 | 0.28 | -0.46 |
| FIX | 0.25 | 64.0 | 43.7 | 1.9 | 0.37 | -0.47 |
| FIX | 0.1 | 81.3 | 53.7 | 2.1 | 0.68 | -0.43 |

| Specimen type | Unit | APTT | InA | InB | *InA | *InB |
|---|---|---|---|---|---|---|
| Heparin | 0.1 | 30.5 | 29.1 | 3.4 | -0.09 | -0.06 |
| Heparin | 0.2 | 36.1 | 27.5 | 3.2 | -0.14 | -0.11 |
| Heparin | 0.3 | 43.5 | 26.0 | 2.9 | -0.19 | -0.21 |
| Heparin | 0.4 | 54.3 | 24.2 | 2.4 | -0.24 | -0.34 |
| Heparin | 0.5 | 64.7 | 23.6 | 2.1 | -0.26 | -0.42 |
| Heparin | 0.6 | 76.9 | 22.4 | 1.9 | -0.30 | -0.49 |
| Heparin | 0.7 | 97.0 | 21.4 | 1.6 | -0.33 | -0.56 |
| Heparin | 0.8 | 104.3 | 21.8 | 1.5 | -0.32 | -0.59 |
| Heparin | 0.9 | 126.3 | 21.2 | 1.4 | -0.34 | -0.61 |
| Heparin | 1.0 | 146.2 | 21.4 | 1.3 | -0.33 | -0.65 |

[Table 3-2]

| Specimen type | APTT | lnA | lnB | *lnA | *lnB |
|---|---|---|---|---|---|
| PNP | 27.9 | 34.9 | 3.6 | 1.7 | 0.1 |
| PNP | 29.9 | 30.8 | 4.0 | -0.6 | 1.4 |
| PNP | 29.1 | 29.9 | 3.5 | -1.1 | -0.5 |
| PNP | 27.6 | 32.4 | 3.3 | 0.3 | -1.4 |
| PNP | 27.5 | 32.6 | 3.8 | 0.4 | 0.8 |
| PNP | 26.1 | 30.8 | 3.5 | -0.6 | -0.4 |

| Specimen type | APTT | lnA | lnB | *lnA | *lnB |
|---|---|---|---|---|---|
| LA | 54.5 | 48.0 | 15.1 | 8.9 | 49.1 |
| LA | 54.1 | 57.3 | 4.1 | 14.1 | 2.0 |
| LA | 53.6 | 56.5 | 3.4 | 13.6 | -0.9 |
| LA | 64.7 | 55.1 | 2.6 | 12.8 | -4.4 |
| LA | 51.5 | 47.7 | 13.8 | 8.7 | 43.7 |
| LA | 68.0 | 52.2 | 2.5 | 11.2 | -5.0 |
| LA | 97.7 | 65.3 | 13.5 | 18.5 | 42.1 |
| LA | 47.2 | 51.2 | 5.9 | 10.7 | 9.6 |
| LA | 54.8 | 46.1 | 14.0 | 7.8 | 44.3 |
| LA | 100.2 | 44.6 | 11.6 | 7.0 | 34.1 |

| Specimen type | APTT | lnA | lnB | *lnA | *lnB |
|---|---|---|---|---|---|
| VIII_Inh | 144.0 | 100.9 | 14.7 | 38.3 | 47.5 |
| VIII_inh | 139.7 | 93.4 | 16.6 | 34.1 | 55.7 |
| VIII_inh | 129.1 | 93.5 | 17.6 | 34.1 | 59.8 |
| VIII_Inh | 156.0 | 98.6 | 14.4 | 36.9 | 46.0 |
| VIII_Inh | 133.6 | 89.4 | 22.0 | 31.8 | 78.7 |
| VIII_Inh | 132.5 | 91.5 | 19.6 | 33.0 | 68.4 |

| Specimen type | Activity | APTT | lnA | lnB | *lnA | *lnB |
|---|---|---|---|---|---|---|
| FVIII | 50 | 32.4 | 35.9 | 4.1 | 2.2 | 2.1 |
| FVIII | 25 | 37.7 | 38.9 | 4.0 | 3.9 | 1.8 |
| FVIII | 10 | 45.5 | 43.7 | 2.8 | 6.6 | -3.3 |
| FVIII | 5 | 51.9 | 49.2 | 3.3 | 9.6 | -1.3 |
| FVIII | 2.5 | 59.7 | 54.4 | 2.8 | 12.4 | -3.7 |
| FVIII | 1 | 70.3 | 61.3 | 2.7 | 16.3 | -3.8 |
| FVIII | 0.75 | 73.1 | 63.8 | 2.7 | 17.7 | -3.8 |
| FVIII | 0.5 | 81.3 | 69.3 | 2.8 | 20.7 | -3.6 |
| FVIII | 0.25 | 89.7 | 76.4 | 3.2 | 24.6 | -1.8 |
| FVIII | 0.1 | 120.0 | 97.4 | 20.2 | 36.3 | 71.1 |

| Specimen type | Activity | APTT | lnA | lnB | *lnA | *lnB |
|---|---|---|---|---|---|---|
| FIX | 50 | 29.0 | 31.5 | 3.5 | -0.2 | -0.4 |
| FIX | 25 | 31.8 | 31.9 | 3.7 | 0.0 | 0.4 |
| FIX | 10 | 36.5 | 33.3 | 3.4 | 0.8 | -0.9 |
| FIX | 5 | 40.9 | 34.0 | 2.5 | 1.2 | -4.6 |
| FIX | 2.5 | 45.6 | 35.2 | 2.2 | 1.8 | -6.1 |
| FIX | 1 | 53.4 | 38.0 | 2.0 | 3.4 | -6.9 |
| FIX | 0.75 | 54.5 | 38.9 | 1.9 | 3.9 | -7.3 |
| FIX | 0.5 | 58.2 | 40.8 | 1.9 | 4.9 | -7.2 |
| FIX | 0.25 | 64.0 | 43.7 | 1.9 | 6.5 | -7.3 |
| FIX | 0.1 | 81.3 | 53.7 | 2.1 | 12.1 | -6.7 |

| Specimen type | Unit | APTT | lnA | lnB | *lnA | *lnB |
|---|---|---|---|---|---|---|
| Heparin | 0.1 | 30.5 | 29.1 | 3.4 | -1.6 | -1.0 |
| Heparin | 0.2 | 36.1 | 27.5 | 3.2 | -2.4 | -1.6 |
| Heparin | 0.3 | 43.5 | 26.0 | 2.9 | -3.3 | -3.3 |
| Heparin | 0.4 | 54.3 | 24.2 | 2.4 | -4.3 | -5.2 |
| Heparin | 0.5 | 64.7 | 23.6 | 2.1 | -4.6 | -6.5 |
| Heparin | 0.6 | 76.9 | 22.4 | 1.9 | -5.3 | -7.5 |
| Heparin | 0.7 | 97.0 | 21.4 | 1.6 | -5.8 | -8.7 |
| Heparin | 0.8 | 104.3 | 21.8 | 1.5 | -5.6 | -9.1 |
| Heparin | 0.9 | 126.3 | 21.2 | 1.4 | -6.0 | -9.5 |
| Heparin | 1.0 | 146.2 | 21.4 | 1.3 | -5.8 | -10.1 |

1.6.4) Change in parameter distribution due to coagulation abnormality

Figure 16 is a diagram showing a trend of a distribution area of a plot of each specimen type on the two-dimensional plot shown in Figure 9C. Distribution areas of plots of SDIs of the pre-CV and the post-CV exhibited different trends in accordance with a specimen type or, in other words, a type of a coagulation abnormality factor of the specimen. However, the distribution area of LA was divided into two of which one overlapped with FVIII.

Figure 17 is a diagram showing a trend of a distribution area of a plot of each specimen type on the two-dimensional plot shown in Figure 11C. Distribution areas of plots of SDIs of the distortion index and the peakedness index exhibited different trends in accordance with a specimen type or, in other words, a type of a coagulation abnormality factor of the specimen. The distribution area of LA was divided into two of which one overlapped with FVIII.

Figure 18 is a diagram showing a trend of a distribution area of a plot of each specimen type on the two-dimensional plot shown in Figure 14C. Distribution areas of plots of SDIs of the pre-post average difference and the pre-post SD ratio exhibited different trends in accordance with a specimen type or, in other words, a type of a coagulation abnormality factor of the specimen.

The results described above suggest that the SDIs of the pre-CV and the post-CV, the SDIs of the distortion index and the peakedness index, or the SDIs of the pre-post average difference and the pre-post SD ratio can be used as indices for estimating a coagulation abnormality factor of a subject specimen. In a similar manner, the relative_ pre-CV and the relative_ post-CV, the relative_ distortion index and the relative_ peakedness index, or the relative_ pre-post average difference and the relative_pre-post SD ratio can also be used as indices for estimating a coagulation abnormality factor of a subject specimen.

First comparative example

A relationship among the parameter Vmax, VmaxT, and a coagulation abnormality was studied using the same specimen group as in the first example. Average values of VmaxT and Vmax were respectively obtained with respect to the six normal specimens (PNP) and adopted as reference_VmaxT and reference_Vmax and, subsequently, relative_VmaxT and relative_Vmax of each specimen were obtained using the following equations.

$$\mathtt{Relative\_VmaxT\ =\ (VmaxT\ of\ subject\ specimen/reference\_VmaxT)\ -\ 1}$$

$$\mathtt{Relative\_Vmax\ =\ (Vmax\ of\ subject\ specimen/reference\_Vmax)\ -\ 1}$$

Figure 19 shows a two-dimensional plot of the relative_VmaxT and the relative_Vmax of each specimen. The plots of all abnormal specimens were positioned at lower right of PNP in accordance with a delay in a coagulation reaction. In addition, positions of the plots exhibited a trend of moving towards the lower right in accordance with a degree of severity (factor concentration or activity) of the specimen. As a result, distribution areas of LA, FVIII, and FIX overlapped with each other and a part of the distribution area of LA overlapped with Heparin.

**Claims**

1. A blood coagulation reaction analysis method, comprising:

    measuring a blood coagulation reaction of a subject specimen and acquiring first data for calculating a blood coagulation time of the subject specimen and second data for estimating a blood coagulation abnormality factor of the subject specimen, wherein
    the acquiring of the second data comprises:

        obtaining a first derivative $V(i)$ of a coagulation reaction curve $R(i)$, where $i$ represents a number of measurement points or time; and
        determining a point $p_k$ where $V(i)$ assumes $X_k$ before reaching a maximum value of $V(i)$, Vmax, and a point $q_k$ where $V(i)$ assumes $X_k$ after reaching Vmax, where $k$ represents a series of integers from 1 to $n$, $n$

denotes an integer equal to or larger than 2, and $0 < X_k < Vmax$.

2. The method according to claim 1, wherein the $X_k$ is specified by $Vmax \times S_k\%$ (wherein $S_k$ ranges from 0.5 to 99).

3. The method according to claim 1 or 2, wherein the acquiring of the second data further comprises calculating $p_k$ or $q_k$ or statistics thereof as the second data.

4. The method according to claim 1 or 2, wherein the acquiring of the second data further comprises calculating, as the second data, at least one selected from the group consisting of a pre-Ave, a post-Ave, a pre-SD, a post-SD, a pre-CV, a post-CV, a pre-post average difference, a pre-post SD ratio, $M_k$, $W_k$, a distortion index, and a peakedness index,

   the pre-Ave, the pre-SD, and the pre-CV respectively representing an average value, a standard deviation, and a coefficient of variation of $p_k$,
   the post-Ave, the post-SD, and the post-CV respectively representing an average value, a standard deviation, and a coefficient of variation of $q_k$,
   the pre-post average difference representing (post-Ave - pre-Ave)/(average value of $p_k$ and $q_k$),
   the pre-post SD ratio representing post-SD/pre-SD,
   $M_k$ representing $(p_k + q_k)/2$,
   $W_k$ representing $q_k - p_k$,
   the distortion index representing a coefficient of variation of $M_k$, and
   the peakedness index representing (sum or average value of $W_k$ with respect to lower part of peak of $V(i)$(sum or average value of $W_k$ with respect to upper part of peak of $V(i)$).

5. The method according to claim 4, wherein the acquiring of the second data further comprises calculating, as the second data, a standard deviation interval (SDI) of an objective parameter with respect to the subject specimen, where

   SDI of objective parameter with respect to the subject specimen = $(\alpha - \beta) \div \gamma$, wherein
   $\alpha$: value of objective parameter from subject specimen
   $\beta$: reference value of value of objective parameter based on data of normal specimen group
   $\gamma$: reference deviation of value of objective parameter based on data of normal specimen group, and
   the objective parameter is any two selected from the group consisting of the pre-CV, the post-CV, the distortion index, the peakedness index, the pre-post average difference, and the pre-post SD ratio.

6. The method according to any one of claims 1 to 5, further comprising calculating a blood coagulation time using the first data.

7. The method according to any one of claims 1 to 6, wherein the first data comprises a point R(E) (where E denotes a coagulation reaction end point) on the coagulation reaction curve R(i) or a maximum value of V(i), Vmax.

8. The method according to any one of claims 1 to 7, comprising acquiring the second data after continuing measurement of the blood coagulation reaction until an end of the coagulation reaction.

9. The method according to any one of claims 1 to 8, further comprising performing estimation of a blood coagulation abnormality factor of the subject specimen based on the second data.

10. The method according to claim 9, wherein the estimation of the blood coagulation abnormality factor comprises estimating a type of the blood coagulation abnormality factor of the subject specimen, and the type of the blood coagulation abnormality factor is selected from the group consisting of a coagulation factor deficiency, lupus anticoagulant-positive, a coagulation factor inhibitor, and heparin-positive.

11. The method according to claim 9 or 10, wherein the estimation of the blood coagulation abnormality factor comprises estimating a presence or absence of the blood coagulation abnormality factor of the subject specimen.

12. The method according to any one of claims 9 to 11, wherein the estimation of the blood coagulation abnormality factor comprises estimating the blood coagulation abnormality factor of the subject specimen in accordance with an estimation model constructed by machine learning,

the estimation model is constructed by machine learning which uses a feature amount representing a blood coagulation reaction of each specimen in a supervised specimen group as an explanatory variable and which uses data with respect to a presence or absence of a coagulation abnormality or to a coagulation abnormality factor of each specimen in the supervised specimen group as a target variable,

the supervised specimen group comprises a blood specimen without a coagulation abnormality and blood specimens with respectively different coagulation abnormality factors,

the feature amount comprises the second data, and

the estimation model estimates a presence or absence of a coagulation abnormality or estimates a coagulation abnormality factor of the subject specimen from the feature amount of the subject specimen.

Fig. 1

START MEASUREMENT

STEP 1 — ACQUIRE REACTION R(i)

STEP 2 — ACQUIRE FIRST DERIVATIVE V(i)

ACQUIRE FIRST DATA — STEP 3

STEP 4 — ACQUIRE SECOND DATA

(ESTIMATE COAGULATION ABNORMALITY FACTOR)    (CALCULATE COAGULATION TIME)

Fig. 2

Fig. 3

Fig. 4

Fig. 5

Fig. 6

Fig. 7

Fig. 8

Fig. 9

Fig. 10

Fig. 11

Fig. 12

Fig. 13

A

B

C

| SPECIMEN TYPE | ACTIVITY OR UNIT | APTT | DISTORTION INDEX | PEAKEDNESS INDEX | *DISTORTION INDEX | *PEAKEDNESS INDEX |
|---|---|---|---|---|---|---|
| FVIII | 50 | 32.4 | 5.7 | 2.3 | 0.09 | -0.10 |
| FVIII | 25 | 37.7 | 5.7 | 2.1 | 0.08 | -0.17 |
| FIX | 50 | 29.0 | 5.0 | 2.4 | -0.05 | -0.04 |
| FIX | 25 | 31.8 | 4.9 | 2.3 | -0.08 | -0.10 |
| FIX | 10 | 36.5 | 4.4 | 2.0 | -0.17 | -0.19 |
| Heparin | 0.1 | 30.5 | 4.6 | 2.5 | -0.12 | 0.00 |
| Heparin | 0.2 | 36.1 | 4.1 | 2.4 | -0.23 | -0.05 |

D

| SPECIMEN TYPE | ACTIVITY OR UNIT | APTT | DISTORTION INDEX | PEAKEDNESS INDEX | *DISTORTION INDEX | *PEAKEDNESS INDEX |
|---|---|---|---|---|---|---|
| FVIII | 50 | 32.4 | 5.7 | 2.3 | 1.3 | -4.2 |
| FVIII | 25 | 37.7 | 5.7 | 2.1 | 1.2 | -7.0 |
| FIX | 50 | 29.0 | 5.0 | 2.4 | -0.8 | -1.7 |
| FIX | 25 | 31.8 | 4.9 | 2.3 | -1.1 | -4.3 |
| FIX | 10 | 36.5 | 4.4 | 2.0 | -2.5 | -7.9 |
| Heparin | 0.1 | 30.5 | 4.6 | 2.5 | -1.8 | 0.0 |
| Heparin | 0.2 | 36.1 | 4.1 | 2.4 | -3.4 | -2.0 |

Fig. 14

Fig. 15

Fig. 16

Fig. 17

Fig. 18

Fig. 19

## INTERNATIONAL SEARCH REPORT

International application No.

**PCT/JP2021/032949**

### A. CLASSIFICATION OF SUBJECT MATTER

*G01N 33/86*(2006.01)i
FI:   G01N33/86

According to International Patent Classification (IPC) or to both national classification and IPC

### B. FIELDS SEARCHED

Minimum documentation searched (classification system followed by classification symbols)

G01N33/86

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | WO 2020/158948 A1 (SEKISUI MEDICAL CO LTD) 06 August 2020 (2020-08-06) claims 1-4, 7-20, paragraphs [0008], [0019]-[0083], [0093]-[0151], fig. 8, 11, 13, 14 | 1-4, 6-11 |
| Y | | 7, 12 |
| A | | 5 |
| X | JP 2019-086517 A (SEKISUI MEDICAL CO LTD) 06 June 2019 (2019-06-06) claim 1, paragraphs [0017]-[0019], [0025], fig. 1-6 | 1-4, 6-11 |
| Y | | 7 |
| Y | JP 2020-056622 A (SYSMEX CORP) 09 April 2020 (2020-04-09) paragraphs [0099]-[0106], fig. 3, 4 | 7 |
| Y | JP 2002-541431 A (AKZO NOBEL N.V) 03 December 2002 (2002-12-03) claims, fig. 1-2 | 12 |
| A | 小田 由紀夫, 凝固波形解析について, 新潟県臨床検査技師会誌, 01 July 2020, vol. 60, no. 3, pp. 205-208 in particular, fig. 5, (ODA, Yukio. Niigata Journal of Medical Technology.), non-official translation (About clot waveform analysis) | 1-12 |

☑ Further documents are listed in the continuation of Box C.   ☑ See patent family annex.

| | | |
|---|---|---|
| * | Special categories of cited documents: | "T"  later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X"  document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&"  document member of the same patent family |
| "P" | document published prior to the international filing date but later than the priority date claimed | |

| Date of the actual completion of the international search | Date of mailing of the international search report |
|---|---|
| **17 November 2021** | **30 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2021/032949** |

**C.      DOCUMENTS CONSIDERED TO BE RELEVANT**

| Category* | Citation of document, with indication, where appropriate, of the relevant passages | Relevant to claim No. |
| --- | --- | --- |
| A | WADA, Hideo et al. Update on the Clot Waveform Analysis. Clinical and Applied Thrombosis/Hemostasis., 30 August 2020, vol. 26, pp. 1-8, DOI:10.1177/1076029620912027 abstract | 1-12 |
| P, X | WO 2020/256107 A1 (SEKISUI MEDICAL CO LTD) 24 December 2020 (2020-12-24) claims | 1-4, 6-12 |
| P, X | SHIMOMURA, Daiki et al. The First-Derivative Curve of the Coagulation Waveform Reveals the Cause of aPTT Prolongation. Clinical and Applied Thrombosis/Hemostasis., 29 December 2020, vol. 26, pp. 1-8, DOI:10.1177/1076029620978810 fig. 1-3 | 1-4, 6-11 |

Form PCT/ISA/210 (second sheet) (January 2015)

## INTERNATIONAL SEARCH REPORT
### Information on patent family members

International application No.

**PCT/JP2021/032949**

| Patent document cited in search report | | | Publication date (day/month/year) | Patent family member(s) | | | Publication date (day/month/year) |
|---|---|---|---|---|---|---|---|
| WO | 2020/158948 | A1 | 06 August 2020 | (Family: none) | | | |
| JP | 2019-086517 | A | 06 June 2019 | (Family: none) | | | |
| JP | 2020-056622 | A | 09 April 2020 | US | 2020/0103420 | A1 | |
| | | | | paragraphs [0134]-[0142], fig. 3-4 | | | |
| | | | | EP | 3629019 | A2 | |
| | | | | CN | 110967507 | A | |
| JP | 2002-541431 | A | 03 December 2002 | US | 2004/0248308 | A1 | |
| | | | | claims, fig. 1-2 | | | |
| | | | | US | 6898532 | B1 | |
| | | | | WO | 2000/046603 | A1 | |
| | | | | EP | 1522860 | A1 | |
| WO | 2020/256107 | A1 | 24 December 2020 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2016194426 A
- JP 2016118442 A
- JP 2017106925 A
- JP 2018017619 A
- JP 2019086517 A
- WO 2020101025 A
- JP 6249855 A
- JP 2019237427 A
- JP 2020039344 A
- JP 2020068877 A

### Non-patent literature cited in the description

- *British Journal of Haematology,* 1997, vol. 98, 68-73
- *The Japanese Journal of Clinical Hematology,* 2017, vol. 58 (9), 1754
- *The Japanese Journal of Thrombosis and Hemostasis,* 2018, vol. 29 (2), 184
- *The Japanese Journal of Thrombosis and Hemostasis,* 2018, vol. 29 (4), 413-420